# EUROPEAN PATENT SPECIFICATION

(11) **EP 2 059 520 B1**
(45) Date of publication and mention of the grant of the patent: **24.02.2010**
(21) Application number: 07826141.9
(22) Date of filing: 27.08.2007
(51) Int. Cl.: C07D 487/04, A61K 31/55, A61P 25/00, A61P 35/00, A61P 9/00

(54) **1,4,5,6,7,8-HEXAHYDRO-I,2,5-TRIAZA-AZULENE DERIVATIVES AS OREXIN RECEPTOR ANTAGONISTS**
1,4,5,6,7,8-HEXAHYDRO-1,2,5-TRIAZA-AZULEN-DERIVATE ALS OREXINREZEPTOR-ANTAGONISTEN
DÉRIVÉS DE 1,4,5,6,7,8-HEXAHYDRO-1,2,5-TRIAZA-AZULÈNE EN TANT QU'ANTAGONISTES DE RÉCEPTEUR D'OREXINE

(30) Priority: 28.08.2006 WO PCT/IB2006/052984
(43) Date of publication of application: 20.05.2009
(73) Proprietor: Actelion Pharmaceuticals Ltd., 4123 Allschwil (CH)
(72) Inventor: AISSAOUI, Hamed, 68840 Pulversheim (FR); BOSS, Christoph, 4123 Allschwil (CH); GUDE, Markus, 4123 Allschwil (CH); KOBERSTEIN, Ralf, 79539 Lörrach (DE); SIFFERLEN, Thierry, 68116 Guewenheim (FR)
(74) Representative: Gschwend, Thomas Peter
(86) International application number: PCT/IB2007/053417
(87) International publication number: WO 2008/026149

(56) References cited:
- WO-A-02/051838
- WO-A-2004/004733

## Description

The present invention relates to novel compounds of formula (II) and their use as pharmaceuticals. The invention also concerns related aspects including processes for the preparation of the compounds, pharmaceutical compositions containing one or more compounds of formula (II), and especially their use as orexin receptor antagonists.

Orexins (orexin A or OX-A and orexin B or OX-B) are novel neuropeptides found in 1998 by two research groups, orexin A is a 33 amino acid peptide and orexin B is a 28 amino acid peptide (Sakurai T. et al., Cell, 1998, 92, 573-585). Orexins are produced in discrete neurons of the lateral hypothalamus and bind to the G-protein-coupled receptors (OX₁ and OX₂ receptors). The orexin-1 receptor (OX₁) is selective for OX-A, and the orexin-2 receptor (OX₂) is capable to bind OX-A as well as OX-B. Orexins are found to stimulate food consumption in rats suggesting a physiological role for these peptides as mediators in the central feedback mechanism that regulates feeding behaviour (Sakurai T. et al., Cell, 1998, 92, 573-585). On the other hand, it was also observed that orexins regulate states of sleep and wakefulness opening potentially novel therapeutic approaches to narcolepsy as well as insomnia and other sleep disorders (Chemelli R.M. et al., Cell, 1999, 98, 437-451).

Orexin receptors are found in the mammalian brain and may have numerous implications in pathologies as known from the literature.

The present invention provides substituted 1,4,5,6,7,8-hexahydro-1,2,5-triaza-azulene derivatives, which are non-peptide antagonists of human orexin OX₁ and

OX₂ receptors. These compounds are in particular of potential use in the treatment of e.g. eating disorders, drinking disorders, sleep disorders, or cognitive dysfunctions in psychiatric and neurologic disorders.

Up to now, some low molecular weight compounds are known having a potential to antagonise either specifically OX₁ or OX₂, or both receptors at the same time. In WO01/85693, Banyu Pharmaceuticals claimed N-acyltetrahydroisoquinoline derivatives. Other orexin receptor antagonists such as novel benzazepine derivatives are disclosed in WO02/051838.

Furthermore, the use of solution-phase chemistry for the lead optimization of 1,2,3,4-tetrahydroisoquinoline derivatives as potential orexin receptor antagonists has been reported (Chimia, 2003, 57, 5, 270-275).

A first aspect of the invention consists of a compound of the general formula II, wherein the chirality is as depicted below, and 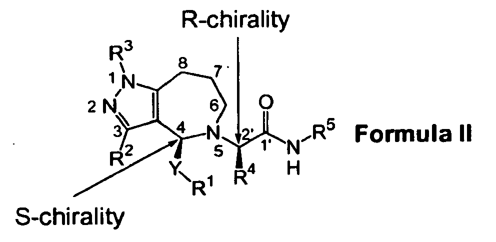 wherein
Y represents -CH₂-CH₂-;
R¹ represents a phenyl group, wherein the phenyl group can be mono-, di-, or trisubstituted, wherein the substituents are independently selected from the group consisting of (C₁₋₄)alkyl, halogen and trifluoromethyl;
R² represents (C₁₋₄)alkyl;
R³ represents (C₁₋₄)alkyl;
R⁴ represents a phenyl group, wherein the phenyl group is unsubstituted or independently mono-, di-, or trisubstituted wherein the substituents are independently selected from the group consisting of (C₁₋₄)alkyl and halogen;
R⁵ represents (C₁₋₄)alkyl.

Also part of the invention are compounds of the formula (II) and pharmaceutically acceptable salts thereof.

In the present description the term "halogen" means fluorine, chlorine or bromine and preferably fluorine or chlorine. In a further preferred embodiment of the invention the term "halogen" means fluorine.

The term "(C₁₋₄)alkyl", alone or in combination, means a straight-chain or branched-chain alkyl group with 1 to 4 carbon atoms. Examples of (C₁₋₄)alkyl groups are methyl, ethyl, propyl, isopropyl, n-butyl, isobutyl, sec.-butyl or tert.-butyl.

For the substituent R² or R⁵ the term "(C₁₋₄)alkyl" preferably means methyl.

For the substituent R³, the term "(C₁₋₄)alkyl" preferably means methyl, ethyl or butyl and more preferably ethyl.

The phenyl group as described in the present invention may be independently mono-, di-, or trisubstituted wherein the substituents are independently selected from the group consisting of (C₁₋₄)alkyl, halogen, and trifluoromethyl. Examples are phenyl, 3,5-difluoro-4-trifluoromethyl-phenyl, dimethyl-phenyl (e.g. 2,3-dimethyl-phenyl, 2,4-dimethyl-phenyl, 3,4-dimethyl-phenyl), 3,5-difluoro-4-methylphenyl, and 2-fluoro-4-trifluoromethyl-phenyl.

R¹ preferably represents a phenyl group which is independently mono-, di-, or trisubstituted (preferred di-, or trisubstituted) wherein the substituents are independently selected from the group consisting of (C₁₋₄)alkyl, halogen, and trifluoromethyl. Preferred, the substituents are independently selected from the group consisting of methyl, ethyl, isopropyl, trifluoromethyl, fluorine and chlorine.

More preferred, the substituents are independently selected from the group consisting of methyl, ethyl, trifluoromethyl, fluorine, and chlorine. Examples are 3,5-difluoro-4-trifluoromethyl-phenyl, dimethyl-phenyl (e.g. 2,3-dimethyl-phenyl, 2,4-dimethyl-phenyl, 3,4-dimethyl-phenyl), 3,5-difluoro-4-methyl-phenyl, and 2-fluoro-4-trifluoromethyl-phenyl.

R⁴ represents a phenyl group which is unsubstituted or independently mono-, di-, or trisubstituted wherein the substituents are independently selected from the group consisting of (C₁₋₄)alkyl, and halogen. For the substituent R⁴ the term "phenyl" preferably means unsubstituted phenyl.

The term "pharmaceutically acceptable salts" refers to non-toxic, inorganic or organic acid and/or base addition salts. Reference can be made to "Salt selection for basic drugs", Int. J. Pharm. (1986), 33, 201-217.

A further preferred embodiment of the invention are compounds of formula II wherein
Y represents -CH₂-CH₂-;
R¹ represents a phenyl group, wherein the phenyl group can be mono-, di-, or trisubstituted, wherein the substituents are independently selected from methyl, ethyl, fluorine, chlorine and trifluoromethyl;
R² represents (C₁₋₄)alkyl;
R³ represents (C₁₋₄)alkyl;
R⁴ represents a phenyl group, wherein the phenyl group is unsubstituted or independently mono-, di-, or trisubstituted wherein the substituents are independently selected from the group consisting of (C₁₋₄)alkyl and halogen;
R⁵ represents (C₁₋₄)alkyl.

A further preferred embodiment of the invention are compounds of formula II
wherein
Y represents -CH₂-CH₂-;
R¹ represents a phenyl group, wherein the phenyl group can be mono-, di-, or trisubstituted, wherein the substituents are independently selected from the group consisting of methyl, ethyl, fluorine, chlorine and trifluoromethyl;
R² represents methyl;
R³ represents ethyl;
R⁴ represents a phenyl group;
R⁵ represents methyl.

The compounds of Formula II may contain one or more stereogenic or asymmetric centers, such as one or more asymmetric carbon atoms. The compounds of Formula II may thus be present as mixtures of stereoisomers or preferably as pure stereoisomers. Mixtures of stereoisomers may be separated in a manner known to a person skilled in the art.

The compounds specifically mentioned above contain two centers of chirality.

In a preferred embodiment, the compounds of the general formula II exhibit the following chirality: (R)-2' and (S)-4 or (R)-2' and (R)-4.

The more preferred stereoisomers exhibit the chirality as depicted below:

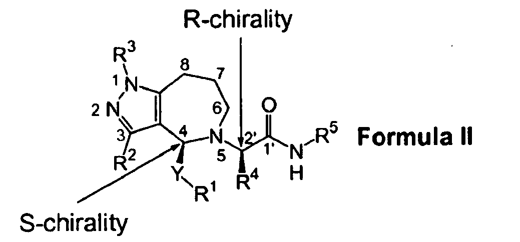

Particularly preferred compounds are listed below:
(R)-2'-{(S)-4-[2-(3,5-difluoro-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamide;
(R)-2'-{(S)-4-[2-(3,4-dimethyl-phenyl)-ethyl]-1-ethyl-3-methyl-4,6,7,8-tetrahydro-1*H*-1,2,5-triaza-azulen-5-yl}-*N*-methyl-2'-phenyl-acetamide;
(R)-2'-{1-ethyl-(S)-4-[2-(2-fluoro-4-trifluoromethyl-phenyl)-ethyl]-3-methyl-4,6,7,8-tetrahydro-1*H*-1,2,5-triaza-azulen-5-yl}-*N*-methyl-2'-phenyl-acetamide;
(R)-2'-{(S)-4-[2-(3,5-difluoro-4-trifluoromethyl-phenyl)-ethyl]-1-ethyl-3-methyl-4,6,7,8-tetrahydro-1*H*-1,2,-5-triaza-azulen-5-yl}-*N*-methyl-2'-phenyl-acetamide;

The compounds of formula II and their pharmaceutically acceptable salts can be used as medicaments, e.g. in the form of pharmaceutical compositions for enteral or parental administration.

The compounds of the general formula II are useful for the treatment and/or prevention of the diseases mentioned herein.

In one embodiment, the invention relates to a method for the treatment and/or prevention of the diseases mentioned herein, said method comprising administering to a subject a pharmaceutically active amount of a compound of general formula II.

The compounds according to formula (II) may be used for the preparation of a medicament, and are suitable, for the prevention or treatment of diseases selected from the group consisting of dysthymic disorders including major depression and cyclothymia, affective neurosis, all types of manic depressive disorders, delirium, psychotic disorders, schizophrenia, catatonic schizophrenia, delusional paranoia, adjustment disorders and all clusters of personality disorders; schizoaffective disorders; anxiety disorders including generalized anxiety, obsessive compulsive disorder, posttraumatic stress disorder, panic attacks, all types of phobic anxiety and avoidance; separation anxiety; all psychoactive substance use, abuse, seeking and reinstatement; all types of psychological or physical addictions, dissociative disorders including multiple personality syndromes and psychogenic amnesias; sexual and reproductive dysfunction; psychosexual dysfunction and addiction; tolerance to narcotics or withdrawal from narcotics; increased anaesthetic risk, anaesthetic responsiveness; hypothalamic-adrenal dysfunctions; disturbed biological and circadian rhythms; sleep disturbances associated with diseases such as neurological disorders including neuropathic pain and restless leg syndrome; sleep apnea; narcolepsy; chronic fatigue syndrome; insomnias related to psychiatric disorders; all types of idiopathic insomnias and parasomnias; sleep-wake schedule disorders including jet-lag; all dementias and cognitive dysfunctions in the healthy population and in psychiatric and neurological disorders; mental dysfunctions of aging; all types of amnesia; severe mental retardation; dyskinesias and muscular diseases; muscle spasticity, tremors, movement disorders; spontaneous and medication-induced dyskinesias; neurodegenerative disorders including Huntington's, Creutzfeld-Jacob's, Alzheimer's diseases and Tourette syndrome; Amyotrophic lateral sclerosis; Parkinson's disease; Cushing's syndrome; traumatic lesions; spinal cord trauma; head trauma; perinatal hypoxia; hearing loss; tinnitus; demyelinating diseases; spinal and cranial nerve diseases; ocular damage; retinopathy; epilepsy; seizure disorders; absence seizures, complex partial and generalized seizures; Lennox-Gastaut syndrome; migraine and headache; pain disorders; anaesthesia and analgesia; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; dental pain; pain related to infection e.g. by HIV; post-chemotherapy pain; post-stroke pain;,post-operative pain; neuralgia; osteoarthritis; conditions associated with visceral pain such as irritable bowel syndrome; eating disorders; diabetes; toxic and dysmetabolic disorders including cerebral anoxia, diabetic neuropathies and alcoholism; appetite, taste, eating, or drinking disorders; somatoform disorders including hypochondriasis; vomiting/nausea; emesis; gastric dyskinesia; gastric ulcers; Kallman's syndrome (anosmia); impaired glucose tolerance; intestinal motility dyskinesias; hypothalamic diseases; hypophysis diseases; hyperthermia syndromes, pyrexia, febrile seizures, idiopathic growth deficiency; dwarfism; gigantism; acromegaly; basophil adenoma; prolactinoma; hyperprolactinemia; brain tumors, adenomas; benign prostatic hypertrophy, prostate cancer; endometrial, breast, colon cancer; all types of testicular dysfunctions, fertility control; reproductive hormone abnormalities; hot flashes; hypothalamic hypogonadism, functional or psychogenic amenorrhea; urinary bladder incontinence; asthma; allergies; all types of dermatitis, acne and cysts, sebaceous gland dysfunctions; cardiovascular disorders; heart and lung diseases, acute and congestive heart failure; hypotension; hypertension; dyslipidemias, hyperlipidemias, insulin resistance; urinary retention; osteoporosis; angina pectoris; myocardial infarction; arrhythmias, coronary diseases, left ventricular hypertrophy; ischemic or haemorrhagic stroke; all types of cerebrovascular disorders including subarachnoid haemorrhage, ischemic and hemorrhagic stroke and vascular dementia; chronic renal failure and other renal diseases; gout; kidney cancer; urinary incontinence; and other diseases related to general orexin system dysfunctions.

The compounds according to formula (II) may be used for the preparation of a medicament, and are suitable, for the prevention or treatment of diseases selected from the group consisting of all types of sleep disorders, of stress-related syndromes, of psychoactive substance use and abuse, of cognitive dysfunctions in the healthy population and in psychiatric and neurologic disorders, of eating or drinking disorders. Eating disorders may be defined as comprising metabolic dysfunction; dysregulated appetite control; compulsive obesities; emeto-bulimia or anorexia nervosa. Pathologically modified food intake may result from disturbed appetite (attraction or aversion for food); altered energy balance (intake vs. expenditure); disturbed perception of food quality (high fat or carbohydrates, high palatability); disturbed food availability (unrestricted diet or deprivation) or disrupted water balance. Drinking disorders include polydipsias in psychiatric disorders and all other types of excessive fluid intake. Sleep disorders include all types of parasomnias, insomnias, narcolepsy and other disorders of excessive sleepiness, sleep-related dystonias; restless leg syndrome; sleep apneas; jet-lag syndrome; shift-work syndrome, delayed or advanced sleep phase syndrome or insomnias related to psychiatric disorders. Insomnias are defined as comprising sleep disorders associated with aging; intermittent treatment of chronic insomnia; situational transient insomnia (new environment, noise) or short-term insomnia due to stress; grief; pain or illness. Insomnia also include stress-related syndromes including post-traumatic stress disorders as well as other types and subtypes of anxiety disorders such as generalized anxiety, obsessive compulsive disorder, panic attacks and all types of phobic anxiety and avoidance; psychoactive substance use, abuse, seeking and reinstatement are defined as all types of psychological or physical addictions and their related tolerance and dependence components. Cognitive dysfunctions include deficits in all types of attention, learning and memory functions occurring transiently or chronically in the normal, healthy, young, adult or aging population, and also occurring transiently or chronically in psychiatric, neurologic, cardiovascular and immune disorders.

In a further preferred embodiment of the invention compounds according to formula (II) may be used for the preparation of a medicament, and are suitable, for the prevention or treatment of diseases selected from the group consisting of sleep disorders that comprises all types of insomnias, narcolepsy and other disorders of excessive sleepiness, sleep-related dystonias, restless leg syndrome, sleep apneas, jet-lag syndrome, shift-work syndrome, delayed or advanced sleep phase syndrome or insomnias related to psychiatric disorders.

In another preferred embodiment of the invention compounds of general formula (II) may be used for the preparation of a medicament, and are suitable, for the prevention or treatment of diseases selected from the group consisting of cognitive dysfunctions that comprise deficits in all types of attention, learning and memory functions occurring transiently or chronically in the normal, healthy, young, adult or aging population, and also occurring transiently or chronically in psychiatric, neurologic, cardiovascular and immune disorders.

In another preferred embodiment of the invention compounds of general formula (II) may be used for the preparation of a medicament, and are suitable, for the prevention or treatment of diseases selected from the group consisting of eating disorders that comprise metabolic dysfunction; dysregulated appetite control; compulsive obesities; emeto-bulimia or anorexia nervosa.

In another preferred embodiment of the invention compounds of general formula (II) may be used for the preparation of a medicament, and are suitable, for the prevention or treatment of diseases selected from the group consisting of psychoactive substance use and abuse that comprise all types of psychological or physical addictions and their related tolerance and dependence components.

The production of the pharmaceutical compositions can be effected in a manner which will be familiar to any person skilled in the art (see for example Mark Gibson, Editor, Pharmaceutical Preformulation and Formulation, IHS Health Group, Englewood, CO, USA, 2001; Remington, The Science and Practice of Pharmacy, 20th Edition, Philadelphia College of Pharmacy and Science; or Remington, The Science and Practice of Pharmacy, 21st Edition (2005), Part 5, "Pharmaceutical Manufacturing" [published by Lippincott Williams & Wilkins]) by bringing the described compounds of Formula (II) and their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

General methods for the preparation of compounds of general formula II: 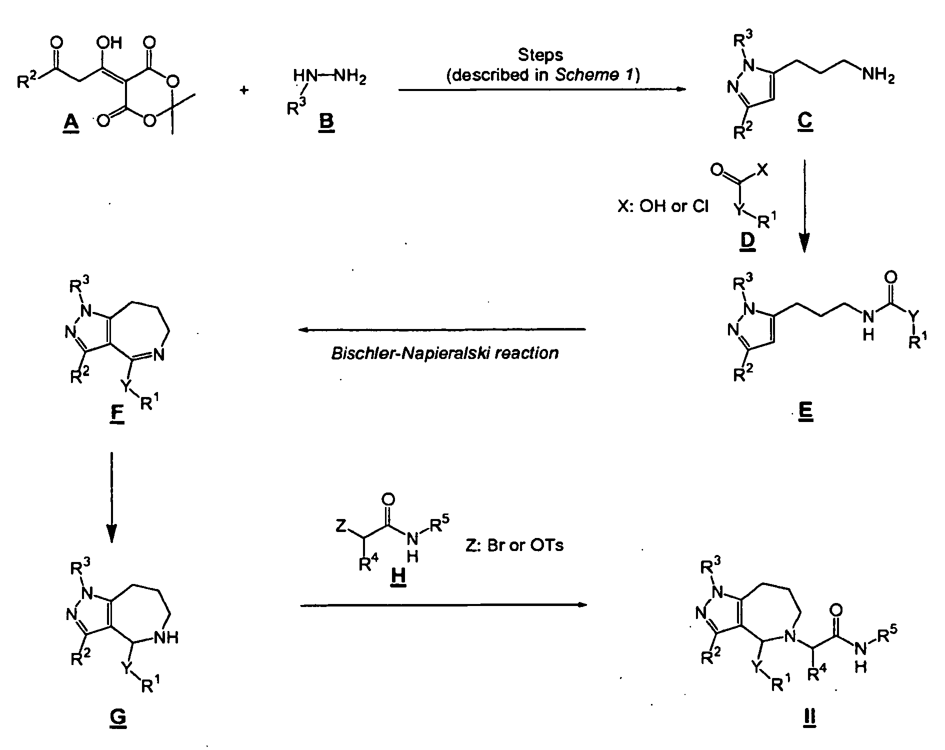 R¹, R², R³, R⁴, R⁵ and Y are as defined in general formula II above, X represents OH or CI and Z represents Br or OTs; The preparation of an example for intermediates **H** is described in WO05/118548.

The acylated *Meldrum's* acid derivative **A** reacted with the mono-substituted hydrazine **B** to give via a multi-step sequence (see *Scheme1* and experimental part) the 3-(2,5-di-substituted-2H-pyrazol-3-yl)-propylamine **C** in a highly regioselective manner. Primary amine **C** was then acylated with carboxylic acid derivative **D** to give the acyclic amide **E** which was transformed into the cyclic seven-membered imine **F** via *Bischler-Napieralski* reaction. Subsequent imine reduction gave the secondary amine precursor **G** which was finally N-alkylated with electrophile **H** in order to obtain the final orexin receptor antagonists **II.** 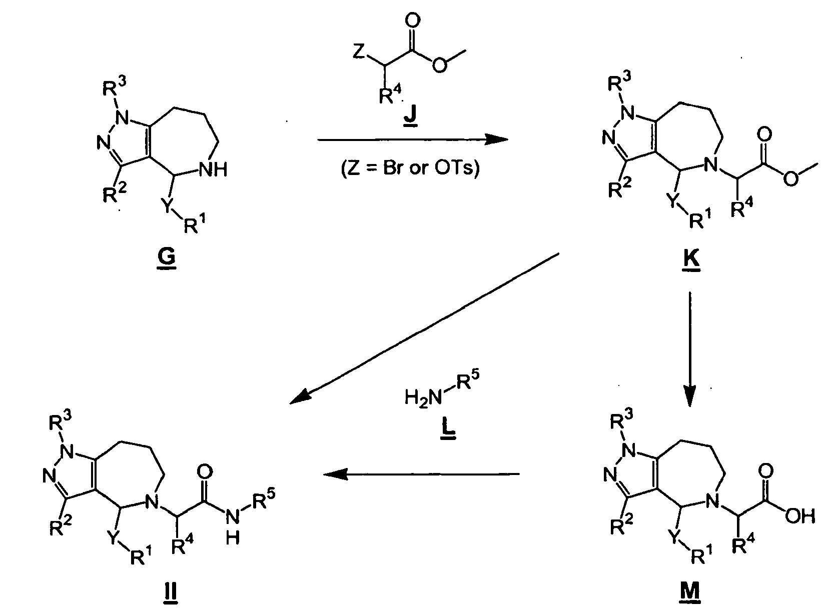 R¹, R², R³, R⁴, R⁵ and Y are as defined in general formula II above, and Z represents Br or OTs; For general methods see also WO 00/168609, WO 02/051838, and WO 04/085403

In a slightly different route the secondary amine **G** could be N-alkylated with ester derivative **J** (instead of amide derivative **H)** to give intermediate **K** which can either be directly transformed into the final compounds **II** by reaction with an amine derivative **L** or which can be first hydrolyzed to the corresponding carboxylic acid **M** followed by reaction with an amine **L** to give final compounds **II.**

In general, all chemical transformations can be performed according to well-known standard methodologies as described in the literature or as described in the procedures below.

### Preparation of compounds of general formula II:

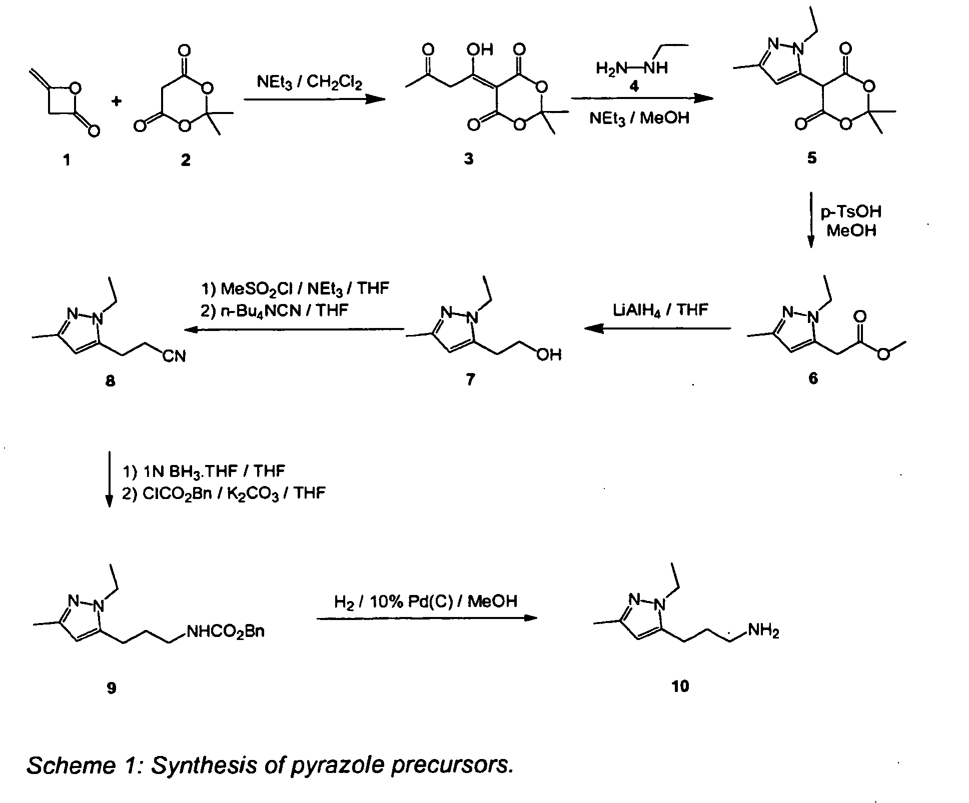

The preparation of 3-(2,5-disubstituted-2*H*-pyrazol-3-yl)-propylamine derivatives (*Scheme 1*) started with the acylation of *Meldrum's* acid **2** with diketene **1** in presence of triethylamine giving intermediate **3.** Reaction of a methanolic solution of **3** with an appropriately substituted hydrazine derivative (e.g. ethylhydrazine **4**) in presence of triethylamine led regioselectively to the formation of the pyrazolo-derivative **5.** Hydrolysis under acidic conditions (p-TsOH in methanol) resulted in the formation of methyl ester **6** which was quantitatively reduced with LiAlH₄ to the corresponding alcohol derivative **7.** Activation of the primary alcohol **7** as the corresponding mesylate and subsequent nucleophilic displacement mediated by tetrabutylammonium cyanide gave the expected nitrile derivative **8.** Borane-mediated reduction of the nitrile **8** followed by protection of the resulting primary amine with a benzyloxycarbonyl moiety gave after flash chromatography the protected compound **9.** Finally, deprotection of the amino function by hydrogenolysis using palladium over charcoal gave the expected primary amine **10.**

In case neither the cinnamic acids nor the hydrocinnamic acids were commercially available, the synthetic routes depicted in *Scheme 2* were followed for their preparation.

### Method A for the preparation of hydrocinnamic acid derivatives from benzaldehyde derivatives via Knoevenagel condensation:

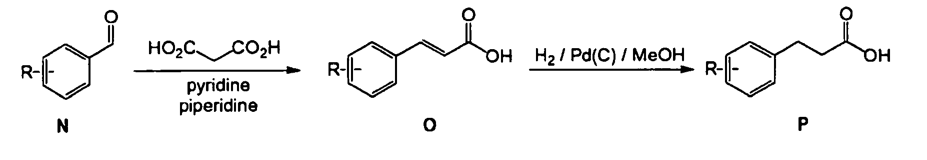

Tho following hydrocinnamic acids were prepared according to this sequence:

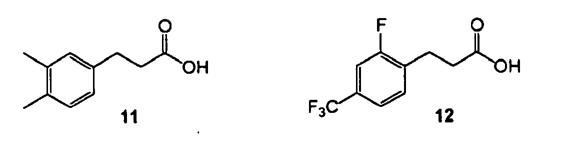

### Method B for the preparation of hydrocinnamic acid derivatives from arylhalides via Heck reaction:

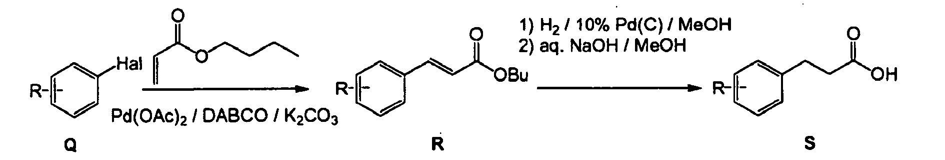

Hal = Br, or I

Tho following hydrocinnamic acids were prepared according to this sequence:

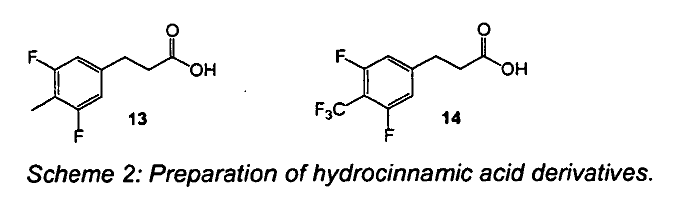

According to "method A", a *Knoevenagel* condensation between benzaldehyde derivatives **N** and malonic acid, in pyridine and in presence of piperidine gave, after acidic work-up, the cinnamic acid derivatives **O.** Catalytic hydrogenation under standard conditions (1 atm H₂; 10% Pd-C; in methanol; rt) led to the corresponding hydrocinnamic acids **P.** Compounds **11** and **12** could be prepared according to this synthetic sequence.

According to "method B", a *Heck* coupling between arylhalide **Q** and butyl acrylate using Pd(OAc)₂/DABCO as a catalytic system gave butyl esters of the expected cinnamic acid derivatives **R.** Subsequent palladium-catalyzed hydrogenation of **R** followed by saponification of the butyl ester led to the target hydrocinnamic acid derivatives **S.** Compounds **13** and **14** could be prepared by following this synthetic route.

*Scheme 3* summarizes the sequence applied for the preparation of final compounds (example in the case of Y = -CH₂CH₂-). For example 3-(2-ethyl-5-methyl-2H-pyrazol-3-yl)-propylamine **10** can be acylated with the hydrocinnamic acid derivative **14** in presence of PyBOP giving the amide derivative **15.** Treatment of **15** under conditions of *Bischler-Napieralski* reaction (POCl₃, in acetonitrile; reflux) led with good yield to the cyclic imine **16** which could be reduced with sodium borohydride to the corresponding secondary amine **17.** S_{N}2-reaction with the tosylate **18** (described in WO 2005/118548) in 2-butanone at 70°C led to an equimolar mixture of diastereoisomers **19.** Finally HPLC-mediated purification allowed the isolation of the pure diastereoisomers **20** [with (S;R) configuration] and **21** [with (R;R) configuration].

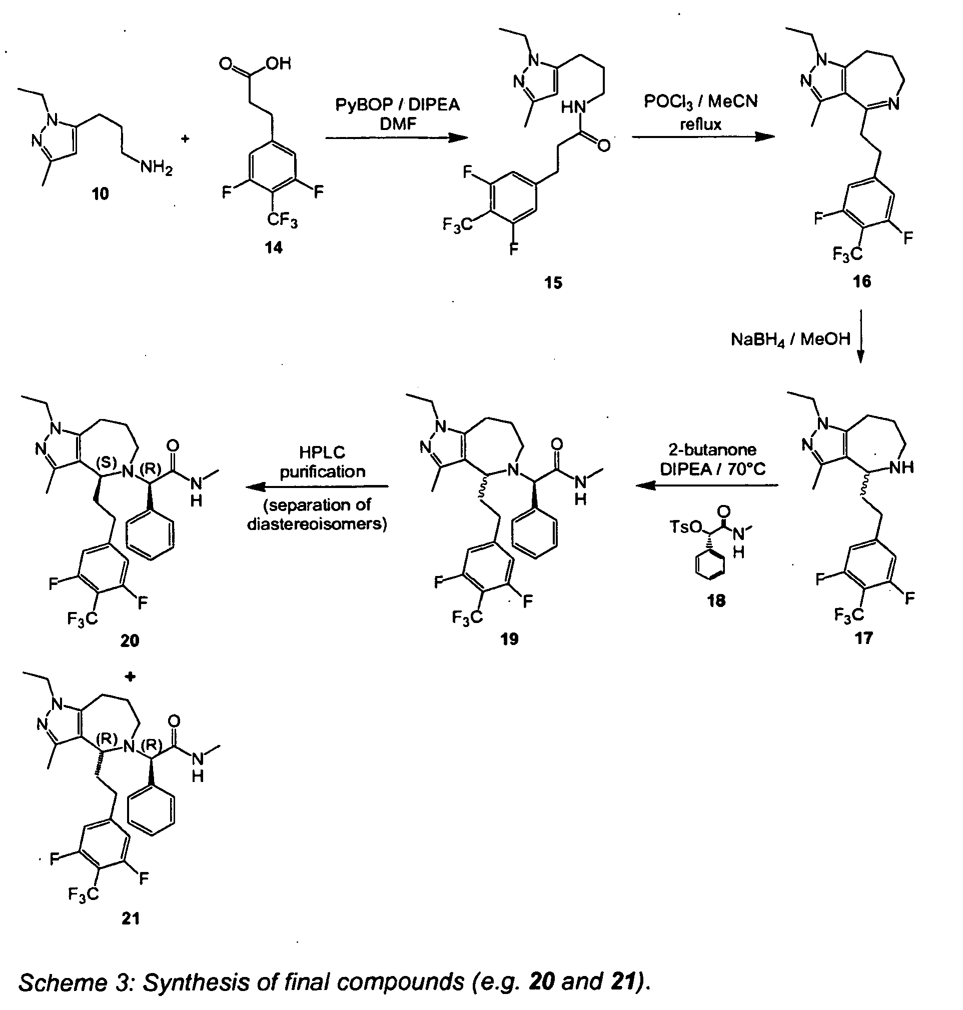

The following examples illustrate the invention but do not limit the scope thereof.

All temperatures are stated in °C.

### Abbreviations (as used herein)

| | |
|---|---|
| AcOEt | ethyl acetate |
| aq. | aqueous |
| BH₃.THF | borane-tetrahydrofuran complex |
| Boc | tert.-butyloxycarbonyl |
| ClCO₂Bn | benzyl chloroformate |
| DABCO | 1,4-diazabicyclo[2.2.2]octane |
| DCM | dichloromethane |
| DIPEA | N-ethyldiisopropylamine |
| DMF | N,N-dimethylformamide |
| DMSO | dimethylsulfoxide |
| EDC | N-(3-dimethylaminopropyl)-N'-ethylcarbodiimide |
| ELSD | Evaporative Light-Scattering Detection |
| Et | ethyl |
| h | hour(s) |
| HPLC | High Performance Liquid Chromatography |
| HV | High Vacuum |
| K₂CO₃ | potassium carbonate |
| LiAlH₄ | lithium aluminum hydride |
| LC-MS | Liquid Chromatography - Mass Spectroscopy |
| MeCN | acetonitrile |
| MeOH | methanol |
| MeSO₂Cl | methanesulfonyl chloride |
| min. | minute(s) |
| MS | Mass Spectroscopy |
| n-Bu₄NCN | tetrabutylammonium cyanide |
| NaBH₄ | sodium borohydride |
| NaOH | sodium hydroxide |
| NEt₃ | triethylamine |
| PBS | phosphate buffered saline |
| Pd(C) | palladium over charcoal |
| Pd(OAc)₂ | palladium (II) acetate |
| POCl₃ | phosphorus oxychloride |
| p-TsOH | para-toluenesulfonic acid |
| PyBOP | benzotriazole-1-yl-oxy-tris-pyrrolidino-phosphonium hexafluorophosphate |
| rt | room temperature |
| sat. | saturated |
| TEA | triethylamine |
| TFA | trifluoroacetic acid |
| THF | tetrahydrofuran |
| TLC | Thin Layer Chromatography |
| t_{R} | retention time |
| UV | ultra violet |
| Vis | visible |

### General Procedures and Examples:

### HPLC conditions:

Analytic: Zorbax 59 SB Aqua column, 4.6 x 50 mm from Agilent Technologies. Eluents: A: acetonitrile; B: H₂O + 0.04% TFA. Gradient: 90% B → 5% B over 2 min. Flow: 4.5 mL/min. Detection: UV/Vis + MS.

Preparative: Waters Xterra RP18 (large), 75 x 30 mm. Eluent: A: Acetonitrile; B: H₂O + 0.05% ammonium hydroxide (25% aq.). Gradient: 90% B → 10% B over 6.5 min. Flow: 75 mUmin. Detection: UV + ELSD.

### Preparation of precursors and intermediates:

### 5-(1-hydroxy-3-oxo-butylidene)-2,2-dimethyl-[1,3]dioxane-4,6-dione

*Meldrum's* acid (50.0 g; 346.9 mmol) was dissolved in DCM (300 ml) and NEt₃ (48.3 ml; 346.9 mmol) was added. The resulting mixture was cooled to 0°C before dropwise addition of diketene (31.9 ml; 416.3 mmol). The reaction mixture was stirred for 2 h at r.t., cooled again to 0°C, and 1 M aqueous hydrochloric acid (700 ml) was slowly added. The layers were separated, the organic layer was dried over anhydrous magnesium sulfate, filtered, and concentrated under reduced pressure to give the product 5-(1-hydroxy-3-oxo-butylidene)-2,2-dimethyl-[1,3]dioxane-4,6-dione as an orange solid which was further dried under HV (79.36 g; 100%). LC-MS: t_{R} = 0.55 min.; [M+H]⁺: no ionisation.

### 5-(2-ethyl-5-methyl-2H-pyrazol-3-yl)-2,2-dimethyl-[1,3]dioxane-4,6-dione

5-(1-Hydroxy-3-oxo-butylidene)-2,2-dimethyl-[1,3]dioxane-4,6-dione (10.0 g; 43.8 mmol) was dissolved in anhydrous methanol (140 ml), and cooled to 0°C before dropwise addition within 15 min. of a solution of ethylhydrazine oxalate (7.24 g; 48.2 mmol) and NEt₃ (13.4 ml; 96.4 mmol) in anhydrous methanol (60 ml). Upon completion of the addition, the mixture was slowly warmed to 60°C and stirring was continued for 90 min. The reaction mixture was concentrated to dryness under reduced pressure, and further dried under HV to give the product 5-(2-ethyl-5-methyl-2H-pyrazol-3-yl)-2,2-dimethyl-[1,3]dioxane-4,6-dione as an orange oil (11.05 g; quantitative yield) which was used in the next step without further purification. LC-MS: t_{R} = 0.52 min.; [M+H]⁺: no ionisation.

### (2-ethyl-5-methyl-2H-pyrazol-3-yl)-acetic acid methyl ester

5-(2-Ethyl-5-methyl-2*H*-pyrazol-3-yl)-2,2-dimethyl-[1,3]dioxane-4,6-dione (11.05 g; 43.8 mmol) was dissolved in anhydrous methanol (125 ml) followed by the addition of p-TsOH x H₂O (36.68 g; 192.85 mmol). The resulting reaction mixture was heated to 60°C for 75 minutes, cooled to 0°C, and triethylamine (26.85 ml; 192.9 mmol) was added dropwise over a period of 20 minutes. The resulting mixture was concentrated under reduced pressure. The residue was taken into DCM (200 ml), washed with water (2 x 75 ml), dried over anhydrous magnesium sulfate, filtered, and concentrated under reduced pressure. Purification by flash chromatography (silicagel; DCM / MeOH = 30 / 1) gave the pure product (2-ethyl-5-methyl-2*H*-pyrazol-3-yl)-acetic acid methyl ester as a yellow oil which was further dried under HV (5.56 g; 70%). LC-MS: t_{R} = 0.62 min.; [M+H]⁺ = 183.18 g/mol.

### 2-(2-ethyl-5-methyl-2H-pyrazol-3-yl)-ethanol

To ice-cooled THF (250 ml) was added in one portion lithium aluminum hydride (2.207 g; 58.171 mmol). The resulting grey suspension was further stirred at 0°C, under nitrogen, and a solution of (2-ethyl-5-methyl-2*H*-pyrazol-3-yl)-acetic acid methyl ester (10.0 g; 54.878 mmol) in anhydrous THF (80 ml) was added dropwise in 30 min. The resulting mixture was stirred at 0°C for 1 h. The ice-cooled reaction mixture was then treated sequentially with water (2.2 ml), 15% aqueous NaOH (2.2 ml), and water (6.6 ml). After additional stirring at rt (30 min.), the resulting precipitate was filtered and washed with diethylether. The resulting filtrate was dried over magnesium sulfate, filtered, concentrated to dryness under reduced pressure, and further dried under HV to give the product 2-(2-ethyl-5-methyl-2*H*-pyrazol-3-yl)-ethanol as a yellow oil (8.45 g; 100%). LC-MS: t_{R} = 0.36 min.; [M+H]⁺ = 155.16 g/mol.

### 3-(2-ethyl-5-methyl-2H-pyrazol-3-yl)-propionitrile

A solution of methanesulfonyl chloride (1.86 ml; 23.950 mmol) in anhydrous THF (10 ml) was added dropwise to an ice-cooled solution of 2-(2-ethyl-5-methyl-2*H-*pyrazol-3-yl)-ethanol (3.358 g, 21.775 mmol) and triethylamine (4.1 ml; 29.390 mmol) in anhydrous THF (100 ml). The resulting mixture was allowed to stir at 0°C, under nitrogen, for 15 min. The reaction mixture was filtered and the solid was washed with anhydrous THF (60 ml). Tetrabutylammonium cyanide (14.611 g; 54.440 mmol) was then added in one portion at rt. The resulting orange solution was heated to 75°C for 1 h. The reaction mixture was allowed to cool to rt before water (30 ml), and diethylether (100 ml) were successively added. After separation, the aqueous layer was further extracted with ethyl acetate (3 x 100 ml). The mixed organic extracts were then dried over anhydrous magnesium sulfate, filtered, and concentrated under reduced pressure. Flash chromatography (silicagel; DCM / MeOH = 40 / 1) gave the pure product 3-(2-ethyl-5-methyl-2*H*-pyrazol-3-yl)-propionitrile as a yellow oil which was further dried under HV (3.07 g; 86%). LC-MS: t_{R} = 0.57 min.; [M+H]⁺ = 164.39 g/mol.

### [3-(2-ethyl-5-methyl-2H-pyrazol-3-yl)-propyl]-carbamic acid benzyl ester

To an ice-cooled solution of 3-(2-ethyl-5-methyl-2*H*-pyrazol-3-yl)-propionitrile (3.070 g; 18.809 mmol) in anhydrous THF (100 ml) was added slowly 1 N BH₃.THF complex solution in THF (75 ml; 75 mmol) The resulting solution was then heated to 75°C for 1h. After cooling to 0°C. aqueous 2N HCl (115 ml; 230 mmol) was carefully added. The resulting solution was stirred at 50°C for 1 h. After cooling to 0°C, solid K₂CO₃ (20.794 g; 150.469 mmol) was added portionwise, followed by dropwise addition of benzyl chloroformate (3.07 ml; 20.689 mmol). The resulting mixture was stirred at 0°C for 1 h, then at rt overnight. Diethylether (100 ml) and water (100 ml) were then added, and the aqueous layer was further extracted with diethylether (2 x 100 ml). The combined organic layers were dried over magnesium sulfate, filtered, and concentrated under reduced pressure. Purification by flash chromatography (silicagel; ethyl acetate / heptane = 1 / 1 => ethyl acetate) gave the pure product [3-(2-ethyl-5-methyl-2*H*-pyrazol-3-yl)-propyl]-carbamic acid benzyl ester as a colorless oil which was further dried under HV (4.21 g; 74%). LC-MS: t_{R} = 0.78 min.; [M+H]⁺ = 302.51 g/mol.

### 3-(2-ethyl-5-methyl-2H-pyrazol-3-yl)-propylamine

A mixture of [3-(2-ethyl-5-methyl-2*H*-pyrazol-3-yl)-propyl]-carbamic acid benzyl ester (1.400 g; 4.645 mmol) and 10% palladium on activated charcoal (280 mg) was placed under nitrogen before methanol (25 ml) was carefully added. The suspension was placed under vacuum, then under hydrogen (1 atm), and the reaction mixture was stirred at rt for 1 h. After filtration over celite, and additional washing of the celite with methanol, the resulting filtrate was concentrated to dryness under reduced pressure. After further drying under HV, the pure product 3-(2-ethyl-5-methyl-2*H*-pyrazol-3-yl)-propylamine (0.774 g; 100%) as a yellow oil could be used for the next step without additional purification. LC-MS: t_{R} = 0.21 min. (broad peak); [M+H]⁺ = 168.39 g/mol.

### 3-(3,4-dimethyl-phenyl)-acrylic acid

A suspension of 3,4-dimethylbenzaldehyde (15.000 g; 111.793 mmol) and malonic acid (22.103 g; 212.410 mmol) in pyridine (85 ml) was heated to 50°C, under nitrogen. Then piperidine (8.5 ml; 86.079 mmol) was added dropwise (over 5 minutes) and the resulting suspension was heated to 75°C for 2h. The reaction mixture was cooled to 0°C, and poured into an ice-cooled solution of concentrated hydrochloric acid (12 N; 96 ml) in water (1200 ml). The precipitated colorless product was filtered off, and washed with water (3 x 100 ml). Remaining water was evaporated under reduced pressure, then under HV to give the dried product 3-(3,4-dimethyl-phenyl)-acrylic acid as a colorless solid (19.23 g; 98%). LC-MS: t_{R} = 0.88 min; [M+H]⁺: no ionisation.

### 3-(3,4-dimethyl-phenyl)-propionic acid

A mixture of 3-(3,4-dimethyl-phenyl)-acrylic acid (19.269 g; 109.355 mmol) and 10% palladium over activated charcoal (1.920 g) was placed under nitrogen before methanol (300 ml) was carefully added. The resulting suspension was placed under vacuum, then under hydrogen (1 atm), and the reaction mixture was vigorously stirred at rt for 4h. The reaction mixture was filtered over a pad of celite, and concentrated under reduced pressure to give the expected product 3-(3,4-dimethyl-phenyl)-propionic acid as a grey solid which was further dried under HV (19.070 g; 98%). LC-MS: t_{R} = 0.85 min; [M+H]⁺: no ionisation.

### 3-(2-fluoro-4-trifluoromethyl-phenyl)-acrylic acid

A suspension of 2-fluoro-4-(trifluoromethyl)benzaldehyde (5.000 g; 26.027 mmol) and malonic acid (5.145 g; 49.451 mmol) in pyridine (20 ml) was heated to 50°C, under nitrogen. Then piperidine (2 ml; 20.040 mmol) was added dropwise (over 5 minutes), and the resulting suspension was heated to 75°C for 3h20. The reaction mixture was cooled to 0°C and poured into an ice-cooled solution of concentrated hydrochloric acid (12 N; 32 ml) in water (400 ml). The precipitated colorless product was filtered off, and washed with water (3 x 100 ml). Remaining water was evaporated under reduced pressure, then under HV to give the dried product 3-(2-fluoro-4-trifluoromethyl-phenyl)-acrylic acid as a colorless solid (5.030 g; 82.5%). LC-MS: t_{R} = 0.89 min; [M+H]⁺: no ionisation.

### 3-(2-fluoro-4-trifluoromethyl-phenyl)-propionic acid

A mixture of 3-(2-fluoro-4-trifluoromethyl-phenyl)-acrylic acid (5.937 g; 25.356 mmol) and 10% palladium over activated charcoal (0.590 g) was placed under nitrogen before methanol (60 ml) was carefully added. The resulting suspension was placed under vacuum, then under hydrogen (1 atm), and the reaction mixture was vigorously stirred at rt for 4h. The reaction mixture was filtered over a pad of celite, and concentrated under reduced pressure to give the expected product 3-(2-fluoro-4-trifluoromethyl-phenyl)-propionic acid as a grey solid which was further dried under HV (4.590 g; 77%). LC-MS: t_{R} = 0.88 min; [M+H]⁺: no ionisation.

### 5-bromo-1,3-difluoro-2-methyl-benzene

A solution of methanesulfonyl chloride (4.72 ml; 60.794 mmol) in anhydrous dichloromethane (10 ml) was added dropwise (over 5 min.) to an ice-cooled solution of 4-bromo-2,6-difluorobenzyl alcohol (11.300 g; 50.669 mmol) and triethylamine (14.1 ml; 101.338 mmol) in dichloromethane (200 ml). The resulting solution was stirred at 0°C, under nitrogen, for 30 min. The reaction mixture was diluted with ethyl acetate (200 ml), and water (100 ml) was added. The organic layer was successively washed with 1N aqueous hydrochloric acid (100 ml), saturated aqueous NaHCO₃ (100 ml), and finally with brine (100 ml). The organic layer was then dried over magnesium sulfate, filtered, and concentrated to dryness under reduced pressure to give an orange solid which was additionally dried under HV (15.170 g; 99.5%). LC-MS for the mesylate: t_{R} = 0.92 min.; [M+H]⁺: no ionisation.

To an ice-cooled solution of the obtained mesylate derivative (15.170 g; 50.381 mmol) in anhydrous THF (90 ml) was added dropwise a solution of superhydride LiEt₃BH (1 N in THF; 106 ml; 106 mmol). The resulting mixture was stirred at 0°C for 5 min., and then at rt for 30 min. The solution was cooled to 0°C before dropwise addition of water (100 ml), and addition of diethylether (200 ml). The organic layer was dried over anhydrous magnesium sulfate, filtered, and carefully concentrated under reduced pressure (CAUTION ! product with low boiling point, therefore heating bath of the rotary evaporator at 30°C !). Purification by flash chromatography (CH₂Cl₂) gave the pure product 5-bromo-1,3-difluoro-2-methylbenzene as a colorless oil (6.910 g; 66%). LC-MS: t_{R} = 1.00 min.; [M+H]⁺: no ionisation.

### 3-(3,5-difluoro-4-methyl-phenyl)-acrylic acid butyl ester

To a solution of 5-bromo-1,3-difluoro-2-methyl-benzene (6.910 g; 33.379 mmol) in anhydrous DMF (200 ml) were added successively butyl acrylate (7.15 ml; 50.062 mmol), DABCO (157 mg; 1.333 mmol), potassium carbonate (4.612 g; 33.379 mmol), and palladium acetate (150 mg; 0.669 mmol). The resulting brown suspension was stirred at 120°C for 1 h. The reaction mixture was allowed to cool to rt before diethylether (400 ml) was added. This mixture was then washed with water (2 x 200 ml), and the mixed aqueous layers were further extracted with diethylether (150 ml). The combined organic layers were dried over anhydrous magnesium sulfate, filtered, and concentrated to dryness under reduced pressure. Purification by flash chromatography (silicagel; DCM / heptane = 1/1 => DCM) gave the pure product 3-(3,5-difluoro-4-methyl-phenyl)-acrylic acid butyl ester as a yellow oil which was further dried under HV (4.69 g; 55%). LC-MS: t_{R} = 1.10 min.; [M+H]⁺: no ionisation.

### 3-(3,5-difluoro-4-methyl-phenyl)-propionic acid butyl ester

A mixture of 3-(3,5-difluoro-4-methyl-phenyl)-acrylic acid butyl ester (7.651 g; 30.089 mmol) and 10% palladium over activated charcoal (0.760 g) was placed under nitrogen before methanol (100 ml) was carefully added. The resulting suspension was placed under vacuum, then under hydrogen (1 atm), and the reaction mixture was vigorously stirred at rt for 2h45. The reaction mixture was filtered over a pad of celite, and concentrated under reduced pressure to give the expected product 3-(3,5-difluoro-4-methyl-phenyl)-propionic acid butyl ester as a yellow oil which was further dried under HV (6.960 g; 90%). LC-MS: t_{R} = 1.10 min; [M+H]⁺: no ionisation.

### 3-(3,5-difluoro-4-methyl-phenyl)-propionic acid

To a solution of 3-(3,5-difluoro-4-methyl-phenyl)-propionic acid butyl ester (6.960 g; 27.157 mmol) in methanol (150 ml) and water (25 ml) was added at rt aqueous 1 N NaOH (68 ml; 68 mmol). The resulting solution was further stirred at rt for 1 h. Methanol was then removed under reduced pressure. Water (25 ml) was added, and the mixture was acidified with aqueous 1 N HCl (68 ml) in order to reach pH = 2. Dichloromethane (150 ml) was added, and the layers were shaken and separated. The aqueous layer was further extracted with dichloromethane (50 ml). The mixed organic layers were dried over anhydrous magnesium sulfate, filtered, and concentrated to dryness under reduced pressure. The product 3-(3,5-difluoro-4-methyl-phenyl)-propionic acid was obtained as a pale yellow solid which was further dried under HV (5.090 g; 94%). LC-MS: t_{R} = 0.86 min.; [M+H]⁺: no ionisation.

### 3-(3,5-difluoro-4-trifluoromethyl-phenyl)-acrylic acid butyl ester

To a solution of 3,5-difluoro-4-(trifluoromethyl)bromobenzene (10.000 g; 38.316 mmol) in anhydrous DMF (235 ml) were added successively butyl acrylate (8.2 ml; 57.474 mmol), DABCO (172 mg; 1.533 mmol), potassium carbonate (5.295 g; 38.316 mmol), and palladium(II) acetate (172 mg; 0.766 mmol). The resulting brown suspension was stirred at 120°C for 2h30. The reaction mixture was allowed to cool to rt before diethylether (500 ml) was added. This mixture was then washed successively with water (1 x 400 ml; 1 x 200 ml) and with brine (50 ml), and the mixed aqueous layers were further extracted with diethylether (300 ml). The combined organic layers were dried over anhydrous magnesium sulfate, filtered, and concentrated to dryness under reduced pressure. Purification by flash chromatography (silicagel; DCM / heptane = 1/1) gave the pure product 3-(3,5-difluoro-4-trifluoromethyl-phenyl)-acrylic acid butyl ester as a slightly beige solid which was further dried under HV (10.120 g; 86%). LC-MS: t_{R} = 1.12 min.; [M+H]⁺: no ionisation.

### 3-(3,5-difluoro-4-trifluoromethyl-phenyl)-propionic acid butyl ester

A mixture of 3-(3,5-difluoro-4-trifluoromethyl-phenyl)-acrylic acid butyl ester (8.849 g; 28.710 mmol) and 10% palladium over activated charcoal (0.885 g) was placed under nitrogen before methanol (120 ml) was carefully added. The resulting suspension was placed under vacuum, then under hydrogen (1 atm), and the reaction mixture was vigorously stirred at rt for 3h30. The reaction mixture was filtered over a pad of celite, and concentrated under reduced pressure to give the expected product 3-(3,5-difluoro-4-trifluoromethyl-phenyl)-propionic acid butyl ester as a yellow oil which was further dried under HV (8.622 g; 97%). LC-MS: t_{R} = 1.11 min; [M+H]⁺: no ionisation.

### 3-(3,5-difluoro-4-trifluoromethyl-phenyl)-propionic acid

To a solution of 3-(3,5-difluoro-4-trifluoromethyl-phenyl)-propionic acid butyl ester (7.658 g; 24.682 mmol) in methanol (168 ml) and water (32 ml) was added at rt aqueous 1 N NaOH (49.4 ml; 49.4 mmol). The resulting solution was further stirred at rt for 45 min. Methanol was then removed under reduced pressure. Water (25 ml) was added, and the mixture was acidified with aqueous 1 N HCl (50 ml) in order to reach pH = 2. Dichloromethane (150 ml) was added, and the layers were shaken and separated. The aqueous layer was further extracted with dichloromethane (50 ml). The mixed organic layers were dried over magnesium sulfate, filtered, and concentrated to dryness under reduced pressure. The product 3-(3,5-difluoro-4-trifluoromethyl-phenyl)-propionic acid was obtained as a colorless solid which was further dried under HV (6.216 g; 99%). LC-MS: t_{R} = 0.90 min.; [M+H]⁺: no ionisation.

### 3-(3,5-difluoro-4-trifluoromethyl-phenyl)-N-[3-(2-ethyl-5-methyl-2H-pyrazol-3-yl)-propyl]-propionamide

To a solution of 3-(3,5-difluoro-4-trifluoromethyl-phenyl)-propionic acid (1.031 g; 4.056 mmol) in anhydrous DMF (17 ml) were added successively PyBOP (2.322 g; 4.462 mmol), a solution of 3-(2-ethyl-5-methyl-2*H*-pyrazol-3-yl)-propylamine (0.678 g; 4.056 mmol) in anhydrous DMF (5 ml), and finally *N-*ethyldiisopropylamine (1.74 ml; 10.140 mmol). The resulting solution was further stirred at rt, under nitrogen, for 1h. Ethyl acetate (100 ml) was added, and the resulting solution was washed with a 1/1 mixture of brine and water (3 x 70 ml). The resulting organic layer was dried over anhydrous magnesium sulfate, filtered, and concentrated to dryness under reduced pressure. The crude orange oil (2.737 g) was purified by flash chromatography (silicagel; DCM / MeOH = 20 / 1) to give the pure product 3-(3,5-difluoro-4-trifluoromethyl-phenyl)-*N*-[3-(2-ethyl-5-methyl-2*H*-pyrazol-3-yl)-propyl]-propionamide as a beige solid which was further dried under HV (1.384 g; 85%). LC-MS: t_{R} = 0.84 min.; [M+H]⁺ = 404.53 g/mol.

### 3-(3,4-dimethyl-phenyl)-N-[3-(2-ethyl-5-methyl-2H-pyrazol-3-yl)-propyl]-propionamide

To a solution of 3-(3,4-dimethyl-phenyl)-propionic acid (0.796 g; 4.470 mmol) in anhydrous DMF (15 ml) were added successively PyBOP (2.558 g; 4.917 mmol), a solution of 3-(2-ethyl-5-methyl-2*H*-pyrazol-3-yl)-propylamine (0.747 g; 4.470 mmol) in anhydrous DMF (10 ml), and finally *N*-ethyldiisopropylamine (1.91 ml; 11.175 mmol). The resulting solution was further stirred at rt, under nitrogen, for 1 h. Ethyl acetate (100 ml) was added, and the resulting solution was washed with a 1/1 mixture of brine and water (3 x 30 ml), and with water (30 ml). The resulting organic layer was dried over anhydrous magnesium sulfate, filtered, and concentrated to dryness under reduced pressure. The crude was purified by flash chromatography (silicagel; DCM / MeOH = 15 / 1) to give the pure product 3-(3,4-dimethyl-phenyl)-*N*-[3-(2-ethyl-5-methyl-2*H*-pyrazol-3-yl)-propyl]-propionamide as a beige solid which was further dried under HV (1.250 g; 85%). LC-MS: t_{R} = 0.82 min.; [M+H]⁺ = 328.24 g/mol.

### N-[3-(2-Ethyl-5-methyl-2H-pyrazol-3-yl)-propyl]-3-(2-fluoro-4-trifluoromethylphenyl)-propionamide

To a solution of 3-(2-fluoro-4-trifluoromethyl-phenyl)-propionic acid (0.776 g; 3.286 mmol) in anhydrous DMF (15 ml) were added successively PyBOP (1.880 g; 3.614 mmol), a solution of 3-(2-ethyl-5-methyl-2*H*-pyrazol-3-yl)-propylamine (0.549 g; 3.286 mmol) in anhydrous DMF (10 ml), and finally N-ethyldiisopropylamine (1.40 ml; 8.215 mmol). The resulting solution was further stirred at rt, under nitrogen, for 1h. Ethyl acetate (100 ml) was added, and the resulting solution was washed with a 1/1 mixture of brine and water (3 x 30 ml).

The resulting organic layer was dried over anhydrous magnesium sulfate, filtered, and concentrated to dryness under reduced pressure. The crude was purified by flash chromatography (silicagel; DCM / MeOH = 15 / 1) to give the pure product *N*-[3-(2-Ethyl-5-methyl-2*H*-pyrazol-3-yl)-propyl]-3-(2-fluoro-4-trifluoromethylphenyl)-propionamide as a beige solid which was further dried under HV (0.950 g; 75%). LC-MS: t_{R} = 0.83 min.; [M+H]⁺ = 386.38 g/mol.

### 3-(3,5-difluoro-4-methyl-phenyl)-N-[3-(2-ethyl-5-methyl-2H-pyrazol-3-yl)-propyl]-propionamide

To a solution of 3-(3,5-difluoro-4-methyl-phenyl)-propionic acid (0.928 g; 4.640 mmol) in anhydrous DMF (15 ml) were added successively PyBOP (2.656 g; 5.104 mmol), a solution of 3-(2-ethyl-5-methyl-2*H*-pyrazol-3-yl)-propylamine (0.776 g; 4.640 mmol) in anhydrous DMF (10 ml), and finally *N-*ethyldiisopropylamine (2.0 ml; 11.600 mmol). The resulting solution was further stirred at rt, under nitrogen, for 1h. Ethyl acetate (100 ml) was added, and the resulting solution was washed with a 1/1 mixture of brine and water (3 x 30 ml), and with water (30 ml). The resulting organic layer was dried over anhydrous magnesium sulfate, filtered, and concentrated to dryness under reduced pressure. The crude was purified by flash chromatography (silicagel; DCM /MeOH = 15 /1) to give the pure product 3-(3,5-difluoro-4-methyl-phenyl)-*N*-[3-(2-ethyl-5-methyl-2*H*-pyrazol-3-yl)-propyl]-propionamide as a colorless solid which was further dried under HV (1.320 g; 81%). LC-MS: t_{R} = 0.81 min.; [M+H]⁺ = 350.53 g/mol.

### 4-[2-(3,5-difluoro-4-trifluoromethyl-phenyl)-ethyl]-1-ethyl-3-methyl-1,4,5,6,7,8-hexahydro-1,2,5-triaza-azulene

A solution of 3-(3,5-difluoro-4-trifluoromethyl-phenyl)-*N*-[3-(2-ethyl-5-methyl-2*H-*pyrazol-3-yl)-propyl]-propionamide (0.919 g; 2.278 mmol) and phosphorus oxychloride (1.05 ml; 11.391 mmol) in anhydrous acetonitrile (25 ml) was heated at reflux (90°C), under nitrogen, for 1 h45. The yellow solution was allowed to cool to room temperature, and was then concentrated to dryness under reduced pressure. The oily residue was dissolved in methanol (5 ml), concentrated again to dryness under reduced pressure, and this co-evaporation with methanol was repeated two additional times (2 x 5 ml MeOH). The resulting crude imine (LC-MS: t R = 0.81 min.; [M+H]⁺: 386.42 g/mol) was reduced to the corresponding amine without additional purification. A solution of this crude imine (0.882 g; 2.278 mmol) in anhydrous methanol (18 ml) was cooled to 0°C before portionwise addition of sodium borohydride (0.431 g; 11.390 mmol). The resulting yellow solution was further stirred at 0°C, under nitrogen, for 4h. After concentration to dryness under reduced pressure, dichloromethane (75 ml), and water (40 ml) were successively added. The aqueous layer was further extracted with dichloromethane (2 x 20 ml). The mixed organic extracts were dried over anhydrous magnesium sulfate, filtered, and concentrated under reduced pressure. The resulting crude (yellow oil; 0.919 g) was purified by flash chromatography (silicagel; DCM / MeOH / 25% aq. NH₄OH = 15 / 1 / 0.1 => DCM / MeOH / aq. NH₄OH = 9 / 1 / 0.1) giving the pure product 4-[2-(3,5-difluoro-4-trifluoromethyl-phenyl)-ethyl]-1-ethyl-3-methyl-1,4,5,6,7,8-hexahydro-1,2,5-triaza-azulene as a beige solid which was further dried under HV (0.473 g; 54%). LC-MS: t_{R} = 0.79 min.; [M+1] = 388.46 g/mol.

### 4-[2-(3,4-dimethyl-phenyl)-ethyl]-1-ethyl-3-methyl-1,4,5,6,7,8-hexahydro-1,2,5-triaza-azulene

A solution of 3-(3,4-dimethyl-phenyl)-*N*-[3-(2-ethyl-5-methyl-2*H*-pyrazol-3-yl)-propyl]-propionamide (1.250 g; 3.817 mmol) and phosphorus oxychloride (1.75 ml; 19.086 mmol) in anhydrous acetonitrile (40 ml) was heated at reflux (90°C), under nitrogen, for 1h15. The resulting solution was allowed to cool to room temperature, and was then concentrated to dryness under reduced pressure. The oily residue was dissolved in methanol (5 ml), concentrated again to dryness under reduced pressure, and this co-evaporation with methanol was repeated two additional times (2 x 5 ml MeOH). The resulting crude imine (yellow oil; LC-MS: t R = 0.76 min.; [M+H]⁺: 310.47 g/mol) was reduced to the corresponding amine without additional purification. A solution of this crude imine (1.181 g; 3.817 mmol) in anhydrous methanol (45 ml) was cooled to 0°C before portionwise addition of sodium borohydride (0.752 g; 19.085 mmol). The resulting solution was further stirred at 0°C, under nitrogen, for 1h15. After concentration to dryness under reduced pressure, dichloromethane (50 ml), and water (50 ml) were successively added. The aqueous layer was further extracted with dichloromethane (25 ml). The mixed organic extracts were dried over anhydrous magnesium sulfate, filtered, and concentrated under reduced pressure. The resulting crude was purified by flash chromatography (silicagel; DCM / MeOH / 25% aq. NH₄OH = 9 / 1 / 0.1) giving the pure product 4-[2-(3,4-dimethyl-phenyl)-ethyl]-1-ethyl-3-methyl-1,4,5,6,7,8-hexahydro-1,2,5-triaza-azulene as a yellow oil which was further dried under HV (0.980 g; 82%). LC-MS: t_{R} = 0.76 min.; [M+1] = 312.51 g/mol.

### 1-ethyl-4-[2-(2-fluoro-4-trifluoromethyl-phenyl)-ethyl]-3-methyl-1,4,5,6,7,8-hexahydro-1,2,5-triaza-azulene

A solution of *N*-[3-(2-ethyl-5-methyl-2*H*-pyrazol-3-yl)-propyl]-3-(2-fluoro-4-trifluoromethyl-phenyl)-propionamide (1.020 g; 2.647 mmol) and phosphorus oxychloride (1.21 ml; 13.233 mmol) in anhydrous acetonitrile (40 ml) was heated at reflux (90°C), under nitrogen, for 1h15. The resulting solution was allowed to cool to room temperature, and was then concentrated to dryness under reduced pressure. The oily residue was dissolved in methanol (5 ml), concentrated again to dryness under reduced pressure, and this co-evaporation with methanol was repeated two additional times (2 x 5 ml MeOH). The resulting crude imine (yellow solid; LC-MS: t R = 0.79 min.; [M+H]⁺: 368.40 g/mol) was reduced to the corresponding amine without additional purification. A solution of this crude imine (0.972 g; 2.647 mmol) in anhydrous methanol (25 ml) was cooled to 0°C before portionwise addition of sodium borohydride (0.521 g; 13.228 mmol). The resulting solution was further stirred at 0°C, under nitrogen, for 1h15. After concentration to dryness under reduced pressure, dichloromethane (50 ml), and water (50 ml) were successively added. The aqueous layer was further extracted with dichloromethane (25 ml). The mixed milky organic extracts were directly concentrated under reduced pressure and the resulting crude was purified by flash chromatography (silicagel; DCM / MeOH / 25% aq. NH₄OH = 9 / 1 / 0.1) giving the pure product 1-ethyl-4-[2-(2-fluoro-4-trifluoromethyl-phenyl)-ethyl]-3-methyl-1,4,5,6,7,8-hexahydro-1,2,5-triaza-azulene as a yellow oil which was further dried under HV (0.453 g; 46%). LC-MS: t_{R} = 0.77 min.; [M+1] = 370.50 g/mol.

### 4-[2-(3,5-difluoro-4-methyl-phenyl)-ethyl]-1-ethyl-3-methyl-1,4,5,6,7,8-hexahydro-1,2,5-triaza-azulene

A solution of 3-(3,5-difluoro-4-methyl-phenyl)-*N*-[3-(2-ethyl-5-methyl-2*H*-pyrazol-3-yl)-propyl]-propionamide (1.320 g; 3.778 mmol) and phosphorus oxychloride (1.73 ml; 18.888 mmol) in anhydrous acetonitrile (40 ml) was heated at reflux (90°C), under nitrogen, for 1h15. The resulting solution was allowed to cool to room temperature, and was then concentrated to dryness under reduced pressure. The oily residue was dissolved in methanol (5 ml), concentrated again to dryness under reduced pressure, and this co-evaporation with methanol was repeated two additional times (2 x 5 ml MeOH). The resulting crude imine (yellow oil; LC-MS: t R = 0.76 min.; [M+H]⁺: 332.46 g/mol) was reduced to the corresponding amine without additional purification. A solution of this crude imine (1.250 g; 3.772 mmol) in anhydrous methanol (45 ml) was cooled to 0°C before portionwise addition of sodium borohydride (0.743 g; 18.859 mmol). The resulting solution was further stirred at 0°C, under nitrogen, for 1h15. After concentration to dryness under reduced pressure, dichloromethane (50 ml), and water (50 ml) were successively added. The aqueous layer was further extracted with dichloromethane (25 ml). The mixed milky organic extracts were directly concentrated under reduced pressure, and the resulting crude was purified by flash chromatography (silicagel; DCM / MeOH / 25% aq. NH₄OH = 9 / 1 / 0.1) giving the pure product 4-[2-(3,5-difluoro-4-methyl-phenyl)-ethyl]-1-ethyl-3-methyl-1,4,5,6,7,8-hexahydro-1,2,5-triaza-azulene as a yellow solid which was further dried under HV (0.958 g; 76%). LC-MS: t_{R} = 0.75 min.; [M+1] = 334.50 g/mol.

### Preparation of final compounds:

### Example 1:

### 2'-{4-[2-(3,5-difluoro-4-trifluoromethyl-phenyl)-ethyl]-1-ethyl-3-methyl-4,6,7,8-tetrahydro-1H-1,2,5-triaza-azulen-5-yl}-N-methyl-2'-phenyl-acetamide

To a solution of 4-[2-(3,5-difluoro-4-trifluoromethyl-phenyl)-ethyl]-1-ethyl-3-methyl-1,4,5,6,7,8-hexahydro-1,2,5-triaza-azulene (0.470 g; 1.213 mmol) in 2-butanone (6 ml) were added successively at rt (S)-toluene-4-sulfonic acid methylcarbamoyl-phenyl-methyl ester (0.426 g; 1.335 mmol), and N-ethyldiisopropylamine (0.41 ml; 2.426 mmol). The reaction mixture was heated to 70°C, under nitrogen, for 23h. Then the reaction mixture was cooled to rt, the solvent was removed under vacuum, dichloromethane (80 ml) was added, and the solution was washed with brine (2 x 80 ml). The organic layer was dried over anhydrous magnesium sulfate, filtered, and concentrated under reduced pressure. Purification by flash chromatography (silicagel; DCM / MeOH = 20/1) gave the mixture of the two epimers (R)-2'-{(S)-4-[2-(3,5-difluoro-4-trifluoromethyl-phenyl)-ethyl]-1-ethyl-3-methyl-4,6,7,8-tetrahydro-1*H*-1,2,5-triaza-azulen-5-yl}-*N*-methyl-2'-phenyl-acetamide and (R)-2'-{(R)-4-[2-(3,5-difluoro-4-trifluoromethyl-phenyl)-ethyl]-1-ethyl-3-methyl-4,6,7,8-tetrahydro-1*H*-1,2,5-triaza-azulen-5-yl}-*N*-methyl-2'-phenyl-acetamide (yellow solid; 0.450 g; 69%). LC-MS: t_{R} = 0.87 min.; [M+H]⁺: 535.57 g/mol.

The optically pure isomers (R)-2'-{(S)-4-[2-(3,5-difluoro-4-trifluoromethyl-phenyl)-ethyl]-1-ethyl-3-methyl-4,6,7,8-tetrahydro-1*H*-1,2,5-triaza-azulen-5-yl}-*N*-methyl-2'-phenyl-acetamide (83 mg; colorless solid; LC-MS: t_{R} = 0.87 min., [M+H]⁺ = 535.57 g/mol) and (R)-2'-{(R)-4-[2-(3,5-difluoro-4-trifluoromethyl-phenyl)-ethyl]-1-ethyl-3-methyl-4,6,7,8-tetrahydro-1*H*-1,2,5-triaza-azulen-5-yl}-*N*-methyl-2'-phenyl-acetamide (75 mg; colorless solid; LC-MS: t_{R} = 0.87 min., [M+H]⁺ = 535.56 g/mol) were then obtained after preparative HPLC-purification of this mixture of epimers.

### Example 2:

### 2'-{4-[2-(3,4-dimethyl-phenyl)-ethyl]-1-ethyl-3-methyl-4,6,7,8-tetrahydro-1H-1,2,5-triaza-azulen-5-yl}-N-methyl-2'-phenyl-acetamide

To a solution of 4-[2-(3,4-dimethyl-phenyl)-ethyl]-1-ethyl-3-methyl-1,4,5,6,7,8-hexahydro-1,2,5-triaza-azulene (0.980 g; 3.146 mmol) in 2-butanone (10 ml) were added successively at rt (S)-toluene-4-sulfonic acid methylcarbamoyl-phenyl-methyl ester (1.105 g; 3.461 mmol), and *N*-ethyldiisopropylamine (1.07 ml; 6.293 mmol). The reaction mixture was heated to 70°C, under nitrogen, for 23h. Then the reaction mixture was cooled to rt, the solvent was removed under vacuum, dichloromethane (80 ml) was added, and the solution was washed with brine (2 x 80 ml). The organic layer was dried over anhydrous magnesium sulfate, filtered, and concentrated under reduced pressure. Purification by flash chromatography (silicagel; DCM / MeOH = 20 / 1) gave the mixture of the two epimers (R)-2'-{(S)-4-[2-(3,4-dimethyl-phenyl)-ethyl]-1-ethyl-3-methyl-4,6,7,8-tetrahydro-1*H*-1,2,5-triaza-azulen-5-yl}-*N*-methyl-2'-phenyl-acetamide and (R)-2'-{(R)-4-[2-(3,4-dimethyl-phenyl)-ethyl]-1-ethyl-3-methyl-4,6,7,8-tetrahydro-1*H-*1,2,5-triaza-azulen-5-yl}-*N*-methyl-2'-phenyl-acetamide (yellow solid; 1.100 g; 76%). LC-MS: t_{R} = 0.84 min.; [M+H]⁺: 459.62 g/mol.

The optically pure isomers (R)-2'-{(S)-4-[2-(3,4-dimethyl-phenyl)-ethyl]-1-ethyl-3-methyl-4,6,7,8-tetrahydro-1*H*-1,2,5-triaza-azulen-5-yl}-*N*-methyl-2'-phenyl-acetamide (76 mg; colorless solid; LC-MS: t_{R} = 0.84 min., [M+H]⁺ = 459.62 g/mol) and (R)-2'-{(R)-4-[2-(3,4-dimethyl-phenyl)-ethyl]-1-ethyl-3-methyl-4,6,7,8-tetrahydro-1*H*-1,2,5-triaza-azulen-5-yl}-*N*-methyl-2'-phenyl-acetamide (70 mg; colorless solid; LC-MS: t_{R} = 0.84 min., [M+H]⁺ = 459.63 g/mol) were then obtained after preparative HPLC-purification of this mixture of epimers.

### Example 3:

### 2'-{1-ethyl-4-[2-(2-fluoro-4-trifluoromethyl-phenyl)-ethyl]-3-methyl-4,6,7,8-tetrahydro-1H-1,2,5-triaza-azulen-5-yl}-N-methyl-2'-phenyl-acetamide

To a solution of 1-ethyl-4-[2-(2-fluoro-4-trifluoromethyl-phenyl)-ethyl]-3-methyl-1,4,5,6,7,8-hexahydro-1,2,5-triaza-azulene (0.453 g; 1.226 mmol) in 2-butanone (5 ml) were added successively at rt (S)-toluene-4-sulfonic acid methylcarbamoyl-phenyl-methyl ester (0.430 g; 1.349 mmol), and *N-*ethyldiisopropylamine (0.42 ml; 2.453 mmol). The reaction mixture was heated to 70°C, under nitrogen, for 24h. Then the reaction mixture was cooled to rt, the solvent was removed under vacuum, dichloromethane (50 ml) was added, and the solution was washed with brine (50 ml). The organic layer was dried over anhydrous magnesium sulfate, filtered, and concentrated under reduced pressure. Purification by flash chromatography (silicagel; DCM / MeOH = 20 / 1) gave the mixture of the two epimers (R)-2'-{1-ethyl-(S)-4-[2-(2-fluoro-4-trifluoromethyl-phenyl)-ethyl]-3-methyl-4,6,7,8-tetrahydro-1*H*-1,2,5-triaza-azulen-5-yl}-*N*-methyl-2'-phenyl-acetamide and (R)-2'-{1-ethyl-(R)-4-[2-(2-fluoro-4-trifluoromethyl-phenyl)-ethyl]-3-methyl-4,6,7,8-tetrahydro-1*H*-1,2,5-triaza-azulen-5-yl}-*N*-methyl-2'-phenyl-acetamide (yellow solid; 0.343 g; 54%). LC-MS: t_{R} = 0.86 min.; [M+H]⁺: 517.62 g/mol.

The optically pure isomers (R)-2'-{1-ethyl-(S)-4-[2-(2-fluoro-4-trifluoromethylphenyl)-ethyl]-3-methyl-4,6,7,8-tetrahydro-1*H*-1,2,5-triaza-azulen-5-yl}-*N*-methyl-2'-phenyl-acetamide (82 mg; colorless solid; LC-MS: t_{R} = 0.86 min., [M+H]⁺ = 517.62 g/mol) and (R)-2'-{1-ethyl-(R)-4-[2-(2-fluoro-4-trifluoromethyl-phenyl)-ethyl]-3-methyl-4,6,7,8-tetrahydro-1*H*-1,2,5-triaza-azulen-5-yl}-*N*-methyl-2'-phenyl-acetamide (90 mg; colorless solid; LC-MS: t_{R} = 0.86 min., [M+H]⁺ = 517.63 g/mol) were then obtained after preparative HPLC-purification of this mixture of epimers.

### Example 4:

### 2'-{4-[2-(3,5-difluoro-4-methyl-phenyl)-ethyl]-1-ethyl-3-methyl-4,6,7,8-tetrahydro-1H-1,2,5-triaza-azulen-5-yl}-N-methyl-2'-phenyl-acetamide

To a solution of 4-[2-(3,5-difluoro-4-methyl-phenyl)-ethyl]-1-ethyl-3-methyl-1,4,5,6,7,8-hexahydro-1,2,5-triaza-azulene (0.958 g; 2.873 mmol) in 2-butanone (10 ml) were added successively at rt (S)-toluene-4-sulfonic acid methylcarbamoyl-phenyl-methyl ester (1.009 g; 3.161 mmol), and N-ethyldiisopropylamine (0.98 ml; 5.746 mmol). The reaction mixture was heated to 70°C, under nitrogen, for 23h. Then the reaction mixture was cooled to rt, the solvent was removed under vacuum, dichloromethane (80 ml) was added, and the solution was washed with brine (2 x 80 ml). The organic layer was dried over anhydrous magnesium sulfate, filtered, and concentrated under reduced pressure. Purification by flash chromatography (silicagel; DCM / MeOH = 20 / 1) gave the mixture of two epimers (R)-2'-{(S)-4-[2-(3,5-difluoro-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamide and (R)-2'-{(R)-4-[2-(3,5-difluoro-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamide (yellow solid; 1.09 g; 79%). LC-MS: t_{R} = 0.84 min.; [M+H]⁺: 481.62 g/mol.

The optically pure isomers (R)-2'-{(S)-4-[2-(3,5-difluoro-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamide (83 mg; colorless solid; LC-MS: t_{R} = 0.84 min., [M+H]⁺ = 481.62 g/mol) and (R)-2'-{(R)-4-[2-(3,5-difluoro-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamide (76 mg; colorless solid; LC-MS: t_{R} = 0.85 min., [M+H]⁺ = 481.64 g/mol) were then obtained after preparative HPLC-purification of this mixture of epimers.

### Biological assays

### In vitro assay

The orexin receptor antagonistic activity of the compounds of formula (II) is determined in accordance with the following experimental method.

### Experimental method:

- Intracellular calcium measurements:
   Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % inactivated fetal calf serum (FCS). The cells are seeded at 80'000 cells / well into 96-well black clear bottom sterile plates (Costar) which have been precoated with 1% gelatine in Hanks' Balanced Salt Solution (HBSS). All reagents are from Gibco BRL. The seeded plates are incubated overnight at 37°C in 5% CO₂.

Human orexin-A as an agonist is prepared as 1 mM stock solution in methanol: water (1:1), diluted in HBSS containing 0.1 % bovine serum albumin (BSA) and 2 mM HEPES for use in the assay at a final concentration of 10 nM.

Antagonists are prepared as 10 mM stock solution in DMSO, then diluted in 96-well plates, first in DMSO, then in HBSS containing 0.1 % bovine serum albumin (BSA) and 2 mM HEPES.

On the day of the assay, 100 µl of loading medium (HBSS containing 1 % FCS, 2 mM HEPES, 5 mM probenecid (Sigma) and 3 µM of the fluorescent calcium indicator fluo-3 AM (1 mM stock solution in DMSO with 10% pluronic acid) (Molecular Probes) is added to each well.

The 96-well plates are incubated for 60 min at 37° C in 5% CO₂. The loading solution is then aspirated and cells are washed 3 times with 200 µl HBSS containing 2.5 mM probenecid, 0.1% BSA, 2 mM HEPES. 100 µl of that same buffer is left in each well.

Within the Fluorescent Imaging Plate Reader (FLIPR, Molecular Devices), antagonists are added to the plate in a volume of 50 µl, incubated for 20 min and finally 100 µl of agonist is added. Fluorescence is measured for each well at 1 second intervals, and the height of each fluorescence peak is compared to the height of the fluorescence peak induced by 10 nM orexin-A with buffer in place of antagonist. For each antagonist, IC₅₀ value (the concentration of compound needed to inhibit 50 % of the agonistic response) is determined. Antagonistic activities of compounds are in the nanomolar range with respect to OX₁ and OX₂ receptors. Selected compounds are displayed in Table 1.

**Table 1:**

| **Product No** | **IC₅₀** OX1 (in nM) | **IC₅₀** OX2 (in nM) |
|---|---|---|
| **(absolute configuration)** | | |
| **Example 1 (4S;2'R)** | **22** | **12** |
| **Example 2 (4S;2'R)** | **36** | **6** |
| **Example 3 (4S;2'R)** | **138** | **18** |
| **Example 4 (4S;2'R)** | **26** | **6** |

## Claims

1. A compound of formula (II), wherein the chirality is as depicted below, and 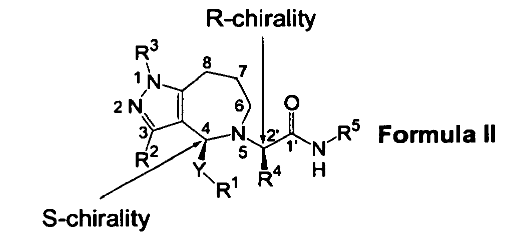 wherein
Y represents -CH₂-CH₂-;
R¹ represents a phenyl group, wherein the phenyl group can be mono-, di-, or trisubstituted, wherein the substituents are independently selected from the group consisting of (C₁₋₄)alkyl, halogen and trifluoromethyl;
R² represents (C₁₋₄)alkyl;
R³ represents (C₁₋₄)alkyl;
R⁴ represents a phenyl group, wherein the phenyl group is unsubstituted or independently mono-, di-, or trisubstituted wherein the substituents are independently selected from the group consisting of (C₁₋₄)alkyl and halogen;
R⁵ represents (C₁₋₄)alkyl;
or a pharmaceutically acceptable salt thereof.

2. A compound according to claim 1, wherein
R¹ represents a phenyl group, wherein the phenyl group can be mono-, di-, or trisubstituted, wherein the substituents are independently selected from methyl, ethyl, fluorine, chlorine and trifluoromethyl;
or a pharmaceutically acceptable salt thereof.

3. A compound according to claim 1 and 2, wherein
R² represents methyl;
R³ represents ethyl;
R⁴ represents a phenyl group;
R⁵ represents methyl;
or a pharmaceutically acceptable salt thereof.

4. A compound according to any one of claims 1 to 3 selected from the group consisting of:
(R)-2'-{(S)-4-[2-(3,5-difluoro-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamide;
(R)-2'-{(S)-4-[2-(3,4-dimethyl-phenyl)-ethyl]-1-ethyl-3-methyl-4,6,7,8-tetrahydro-1*H*-1,2,5-triaza-azulen-5-yl}-*N*-methyl-2'-phenyl-acetamide;
(R)-2'-{1-ethyl-(S)-4-[2-(2-fluoro-4-trifluoromethyl-phenyl)-ethyl]-3-methyl-4,6,7,8-tetrahydro-1*H*-1,2,5-triaza-azulen-5-yl}-*N*-methyl-2'-phenyl-acetamide; and
(R)-2'-{(S)-4-[2-(3,5-difluoro-4-trifluoromethyl-phenyl)-ethyl]-1-ethyl-3-methyl-4,6,7,8-tetrahydro-1*H*-1,2,5-triaza-azulen-5-yl}-*N*-methyl-2'-phenyl-acetamide;
or a pharmaceutically acceptable salt thereof.

5. A compound according to anyone of claims 1 to 4 for use as a medicament.

6. Use of a compound according to any of claims 1 to 4 for the preparation of a medicament for the prevention or treatment of diseases selected from the group consisting of dysthymic disorders including major depression and cyclothymia, affective neurosis, all types of manic depressive disorders, delirium, psychotic disorders, schizophrenia, catatonic schizophrenia, delusional paranoia, adjustment disorders and all clusters of personality disorders; schizoaffective disorders; anxiety disorders including generalized anxiety, obsessive compulsive disorder, posttraumatic stress disorder, panic attacks, all types of phobic anxiety and avoidance; separation anxiety; all psychoactive substance use, abuse, seeking and reinstatement; all types of psychological or physical addictions, dissociative disorders including multiple personality syndromes and psychogenic amnesias; sexual and reproductive dysfunction; psychosexual dysfunction and addiction; tolerance to narcotics or withdrawal from narcotics; increased anaesthetic risk, anaesthetic responsiveness; hypothalamic-adrenal dysfunctions; disturbed biological and circadian rhythms; sleep disturbances associated with diseases such as neurological disorders including neuropathic pain and restless leg syndrome; sleep apnea; narcolepsy; chronic fatigue syndrome; insomnias related to psychiatric disorders; all types of idiopathic insomnias and parasomnias; sleep-wake schedule disorders including jet-lag; all dementias and cognitive dysfunctions in the healthy population and in psychiatric and neurological disorders; mental dysfunctions of aging; all types of amnesia; severe mental retardation; dyskinesias and muscular diseases; muscle spasticity, tremors, movement disorders; spontaneous and medication-induced dyskinesias; neurodegenerative disorders including Huntington's, Creutzfeld-Jacob's, Alzheimer's diseases and Tourette syndrome; Amyotrophic lateral sclerosis; Parkinson's disease; Cushing's syndrome; traumatic lesions; spinal cord trauma; head trauma; perinatal hypoxia; hearing loss; tinnitus; demyelinating diseases; spinal and cranial nerve diseases; ocular damage; retinopathy; epilepsy; seizure disorders; absence seizures, complex partial and generalized seizures; Lennox-Gastaut syndrome; migraine and headache; pain disorders; anaesthesia and analgesia; enhanced or exaggerated sensitivity to pain such as hyperalgesia, causalgia, and allodynia; acute pain; bum pain; atypical facial pain; neuropathic pain; back pain; complex regional pain syndrome I and II; arthritic pain; sports injury pain; dental pain; pain related to infection e.g. by HIV; post-chemotherapy pain; post-stroke pain; post-operative pain; neuralgia; osteoarthritis; conditions associated with visceral pain such as irritable bowel syndrome; eating disorders; diabetes; toxic and dysmetabolic disorders including cerebral anoxia, diabetic neuropathies and alcoholism; appetite, taste, eating, or drinking disorders; somatoform disorders including hypochondriasis; vomiting/nausea; emesis; gastric dyskinesia; gastric ulcers; Kallman's syndrome (anosmia); impaired glucose tolerance; intestinal motility dyskinesias; hypothalamic diseases; hypophysis diseases; hyperthermia syndromes, pyrexia, febrile seizures, idiopathic growth deficiency; dwarfism; gigantism; acromegaly; basophil adenoma; prolactinoma; hyperprolactinemia; brain tumors, adenomas; benign prostatic hypertrophy, prostate cancer; endometrial, breast, colon cancer; all types of testicular dysfunctions, fertility control; reproductive hormone abnormalities; hot flashes; hypothalamic hypogonadism, functional or psychogenic amenorrhea; urinary bladder incontinence; asthma; allergies; all types of dermatitis, acne and cysts, sebaceous gland dysfunctions; cardiovascular disorders; heart and lung diseases, acute and congestive heart failure; hypotension; hypertension; dyslipidemias, hyperlipidemias, insulin resistance; urinary retention; osteoporosis; angina pectoris; myocardial infarction; arrhythmias, coronary diseases, left ventricular hypertrophy; ischemic or haemorrhagic stroke; all types of cerebrovascular disorders including subarachnoid haemorrhage, ischemic and hemorrhagic stroke and vascular dementia; chronic renal failure and other renal diseases; gout; kidney cancer; urinary incontinence; and other diseases related to general orexin system dysfunctions.

7. Use of a compound according to any of claims 1 to 4 for the preparation of a medicament for the prevention or treatment of a disease selected from the group consisting of all types of sleep disorders, of stress-related syndromes, of psychoactive substance use and abuse, of cognitive dysfunctions in the healthy population and in psychiatric and neurologic disorders, of eating or drinking disorders.

## Patentansprüche

1. Verbindung der Formel (II), wobei die Chiralität wie nachfolgend dargestellt ist und 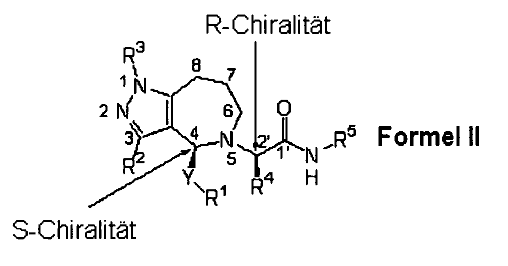 wobei
Y -CH₂-CH₂- repräsentiert,
R¹ eine Phenylgruppe repräsentiert, wobei die Phenylgruppe mono-, di- oder trisubstituiert sein kann, wobei die Substituenten unabhängig ausgewählt sind aus der Gruppe bestehend aus (C₁₋₄)-Alkyl, Halogen und Trifluormethyl,
R² (C₁₋₄)-Alkyl repräsentiert,
R³ (C₁₋₄)-Alkyl repräsentiert,
R⁴ eine Phenylgruppe repräsentiert, wobei die Phenylgruppe nicht substituiert ist oder unabhängig mono-, di- oder trisubstituiert ist, wobei die Substituenten unabhängig ausgewählt sind aus der Gruppe bestehend aus (C₁₋₄)-Alkyl und Halogen,
R⁵ (C₁₋₄)-Alkyl repräsentiert,
oder ein pharmazeutisch akzeptables Salz davon.

2. Verbindung nach Anspruch 1, wobei
R¹ eine Phenylgruppe repräsentiert, wobei die Phenylgruppe mono-, di- oder trisubstituiert sein kann, wobei die Substituenten unabhängig ausgewählt sind aus Methyl, Ethyl, Fluor, Chlor und Trifluormethyl,
oder ein pharmazeutisch akzeptables Salz davon.

3. Verbindung nach Anspruch 1 und 2, wobei
R² Methyl repräsentiert,
R³ Ethyl repräsentiert,
R⁴ eine Phenylgruppe repräsentiert,
R⁵ Methyl repräsentiert,
oder ein pharmazeutisch akzeptables Salz davon.

4. Verbindung nach einem der Ansprüche 1 bis 3, die ausgewählt sind aus der Gruppe bestehend aus:
(R)-2'-{(S)-4-[2-(3,5-Difluor-4-methyl-phenyl)-ethyl]-1-ethyl-3-methyl-4,6,7,8-tetrahydro-1*H*-1,2,5-triaza-azulen-5-yl}-*N*-methyl-2'-phenyl-acetamid;
(R)-2'-{(S)-4-[2-(3,4-Dimethyl-phenyl)-ethyl]-1-ethyl-3-methyl-4,6,7,8-tetrahydro-1*H*-1,2,5-triaza-azulen-5-yl}-*N*-methyl-2'-phenyl-acetamid;
(R)-2'-{1-Ethyl-(S)-4-[2-(2-fluor-4-trifluormethyl-phenyl)-ethyl]-3-methyl-4,6,7,8-tetrahydro-1*H*-1,2,5-triaza-azulen-5-yl}-*N*-methyl-2'-phenyl-acetamid;
und
(R)-2'-{(S)-4-[2-(3,5-Difluor-4-trifluormethyl-phenyl)-ethyl]-1-ethyl-3-methyl-4,6,7,8-tetrahydro-1*H*-1,2,5-triaza-azulen-5-yl}-*N*-methyl-2'-phenyl-acetamid;
oder ein pharmazeutisch akzeptables Salz davon.

5. Verbindung nach einem der Ansprüche 1 bis 4 zur Verwendung als Medikament.

6. Verwendung einer Verbindung nach einem der Ansprüche 1 bis 4 zur Herstellung eines Medikaments zur Vorbeugung gegen oder Behandlung von Krankheiten, die ausgewählt sind aus der Gruppe bestehend aus dysthymischen Störungen wie Major Depression und Zyklothymie, affektive Neurose, alle Arten von manisch-depressiven Störungen, Delirium, psychotische Störungen, Schizophrenie, katatonische Schizophrenie, wahnhafte Paranoia, Anpassungsstörungen und alle Cluster von Persönlichkeitsstörungen; schizoaffektiven Störungen; Angststörungen wie generalisierte Angst, obsessive Zwangsstörung, posttraumatische Belastungsstörung, Panikattacken, alle Arten von phobischer Angst und Vermeidung; Trennungsangst; jeglichem/r Psychotropika-Gebrauch, -Missbrauch, -Sucht und -Reinstatement; allen Arten der psychologischen oder physischen Abhängigkeit, dissoziativen Störungen wie multiple Persönlichkeitssyndrome und psychogene Amnesien; sexueller und reproduktiver Dysfunktion; psychosexueller Dysfunktion und Abhängigkeit; Toleranz gegenüber Narkotika oder Entzug von Narkotika; erhöhtem Anästhesierisiko, Anästhesieresponsivität; hypothalamisch-adrenalen Dysfunktionen; gestörten biologischen und circadianen Rhythmen; Schlafstörungen in Verbindung mit Krankheiten wie z.B. neurologische Störungen wie neuropathische Schmerzen und Restless-Leg-Syndrom; Schlafapnoe; Narkolepsie; chronischem Erschöpfungssyndrom; durch psychiatrische Störungen bedingten Insomnien; allen Arten von idiopathischen Insomnien und Parasomnien; Wach-Schlafrhythmusstörungen wie Jet-Lag; jeglichen Demenzen und kognitiven Dysfunktionen bei der gesunden Bevölkerung und bei psychiatrischen und neurologischen Störungen; altersbedingten mentalen Dysfunktionen; allen Arten von Amnesie; starker mentaler Retardierung; Dyskinesien und Muskelerkrankungen; Muskelspastik, Tremoren, Bewegungsstörungen; spontanen und medikamenteninduzierten Dyskinesien; neurodegenerativen Störungen wie die Huntington-, Creutzfeld-Jakob-, Alzheimer-Krankheit und das Tourette-Syndrom; amyotropher Lateralsklerose; der Parkinson-Krankheit; dem Cushing-Syndrom; traumatischen Läsionen; Rückenmarktrauma; Kopftrauma; perinataler Hypoxie; Hörverlust; Tinnitus; Entmarkungskrankheiten; spinalen und kranialen Nervenkrankheiten; Augenschaden; Retinopathie; Epilepsie; Anfallsstörungen; Absence-Anfällen, komplexen partiellen und generalisierten Anfällen; Lennox-Gastaut-Syndrom; Migräne und Kopfschmerzen; Schmerzstörungen; Anästhesie und Analgesie; erhöhter oder übertriebener Empfindlichkeit gegenüber Schmerz wie Hyperalgesie, Kausalgie und Allodynie; akuten Schmerzen; Verbrennungsschmerzen; atypischen Gesichtsschmerzen; neuropathischen Schmerzen; Rückenschmerzen; komplexem regionalem Schmerzsyndrom I und II; arthritischen Schmerzen; Sportverletzungsschmerzen; Zahnschmerzen; durch eine Infektion bedingten Schmerzen, z.B. durch HIV; Post-Chemotherapie-Schmerzen; Post-Hirnschlag-Schmerzen; postoperativen Schmerzen; Neuralgie; Osteoarthritis; Zuständen in Verbindung mit viszeralen Schmerzen wie Reizdarmsyndrom; Essstörungen; Diabetes; toxischen und dysmetabolischen Störungen wie zerebrale Anoxie, diabetische Neuropathien und Alkoholismus; Appetit-, Geschmacks-, Ess- oder Trinkstörungen; somatoformen Störungen wie Hypochondrie; Erbrechen/Übelkeit; Emesis; gastrischer Dyskinesie; Magengeschwüren; Kallman-Syndrom (Anosmie); gestörter Glucosetoleranz; Darmmotilitätsdyskinesien; Hypothalamuskrankheiten; Hypophysekrankheiten; Hyperthermiesyndromen, Pyrexie, Fieberanfällen, idiopathischer Wachstumsdefizienz; Kleinwuchs; Riesenwuchs; Akromegalie; basophilem Adenom; Prolaktinom; Hyperprolaktinämie; Gehirntumoren, Adenomen; benigner Prostatahypertrophie, Prostatakrebs; Endometrium-, Brust-, Darmkrebs; allen Arten von Hodendysfunktionen, Fertilitätskontrolle; reproduktiven Hormonanomalien; Hitzewallungen; hypothalamischem Hypogonadismus, funktioneller oder psychogener Amenorrhoe; Harnblaseninkontinenz; Asthma; Allergien; allen Arten von Dermatitis, Akne und Zysten, Talgdrüsendysfunktionen; Herz-Kreislauf-Störungen; Herz- und Lungen-Krankheiten, akuter und kongestiver Herzinsuffizienz; Hypotonie; Hypertonie; Dyslipidämie, Hyperlipidämie, Insulinresistenz; Harnverhaltung; Osteoporose; Angina pectoris; Myokardinfarkt; Arrhythmien, koronaren Krankheiten, linksventrikulärer Hypertrophie; ischämischem oder hämorrhagischem Schlaganfall; allen Arten von zerebrovaskulären Störungen wie Subarachnoidalblutung, ischämischer und hämorrhagischer Schlaganfall und vaskuläre Demenz; chronischem Nierenversagen und anderen Nierenkrankheiten; Gicht; Nierenkrebs; Harninkontinenz; und anderen Krankheiten in Verbindung mit allgemeinen Orexinsystemdysfunktionen.

7. Verwendung einer Verbindung nach einem der Ansprüche 1 bis 4 zur Herstellung eines Medikaments zur Vorbeugung gegen oder Behandlung von Krankheiten, die ausgewählt sind aus der Gruppe bestehend aus allen Arten von Schlafstörungen, von stressbedingten Syndromen, von Psychotropika-Gebrauch und -Missbrauch, von kognitiven Dysfunktionen bei der gesunden Bevölkerung und bei psychiatrischen und neurologischen Störungen, von Ess- oder Trinkstörungen.

## Revendications

1. Composé de formule (II), où la chiralité est telle qu'illustrée ci-dessous, et 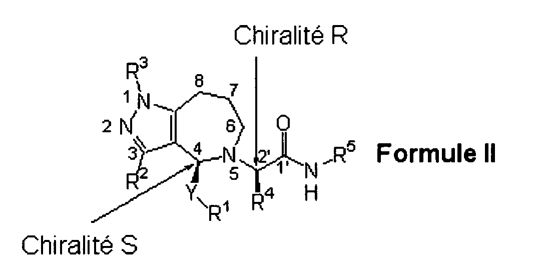 où
Y représente -CH₂-CH₂- ;
R¹ représente un groupement phényle, où le groupement phényle peut être mono-, di- ou trisubstitué par des substituants qui sont indépendamment sélectionnés dans le groupe consistant en un alkyle en (C₁₋₄), un halogène et un trifluorométhyle ;
R² représente un alkyle en (C₁₋₄) ;
R³ représente un alkyle en (C₁₋₄) ;
R⁴ représente un groupement phényle, où le groupement phényle est non substitué ou est indépendamment mono-, di- ou trisubstitué par des substituants qui sont indépendamment sélectionnés dans le groupe consistant en un alkyle en (C₁₋₄) et un halogène ;
R⁵ représente un alkyle en (C₁₋₄) ;
ou sel pharmaceutiquement acceptable de celui-ci.

2. Composé selon la revendication 1, où
R¹ représente un groupement phényle, où le groupement phényle peut être mono-, di- ou trisubstitué par des substituants qui sont indépendamment sélectionnés dans le groupe consistant en un méthyle, un éthyle, un fluor, un chlore et un trifluorométhyle ;
ou sel pharmaceutiquement acceptable de celui-ci.

3. Composé selon la revendication 1 et 2, où
R² représente un méthyle ;
R³ représente un éthyle ;
R⁴ représente un groupement phényle ;
R⁵ représente un méthyle ;
ou sel pharmaceutiquement acceptable de celui-ci.

4. Composé selon l'une quelconque des revendications 1 à 3, sélectionné dans le groupe consistant en les suivants :
(R)-2'-{(S)-4-[2-(3,5-difluoro-4-méthyl-phényl)-éthyl]-1-éthyl-3-méthyl-4,6,7,8-tétrahydro-1*H*-1,2,5-triaza-azulèn-5-yl}-*N*-méthyl-2'-phényl-acétamide ;
(R)-2'-{(S)-4-[2-(3,4-diméthyl-phényl)-éthyl]-1-éthyl-3-méthyl-4,6,7,8-tétrahydro-1*H*-1,2,5-triaza-azulèn-5-yl}-*N*-méthyl-2'-phényl-acétamide ;
(R)-2'-{1-éthyl-(S)-4-[2-(2-fluoro-4-trifluorométhyl-phényl)-éthyl]-3-méthyl-4,6,7,8-tétrahydro-1*H*-1,2,5-triaza-azulèn-5-yl}-*N*-méthyl-2'-phényl-acétamide ;
et
(R)-2'-{(S)-4-[2-(3,5-difluoro-4-trifluorométhyl-phényl)-éthyl]-1-éthyl-3-méthyl-4,6,7,8-tétrahydro-1*H*-1,2,5-triaza-azulèn-5-yl}-*N*-méthyl-2'-phényl-acétamide ;
ou sel pharmaceutiquement acceptable de celui-ci.

5. Composé selon l'une quelconque des revendications 1 à 4, qui est utilisé comme médicament.

6. Utilisation d'un composé selon l'une quelconque des revendications 1 à 4 dans la préparation d'un médicament indiqué dans la prévention ou le traitement d'affections sélectionnées dans le groupe consistant en les suivantes : états dysthymiques, y compris dépression majeure et cyclothymie, névrose affective, tous les types de troubles maniacodépressifs, délire, troubles psychotiques, schizophrénie, schizophrénie catatonique, paranoïa délirante, troubles de l'adaptation et tous les groupes de troubles de la personnalité ; troubles schizo-affectifs ; troubles anxieux, y compris anxiété généralisée, trouble obsessionnel compulsif, syndrome de stress post-traumatique, attaques de panique, tous les types d'anxiété et d'évitement phobiques ; névrose d'abandon ; usage, abus, comportement de recherche et récidive d'usage de toute substance psychoactive ; tous les types d'addiction psychologique ou physique, troubles dissociatifs, y compris syndromes de la personnalité multiple et amnésies psychogènes ; dysfonction sexuelle et troubles de la fonction reproductive ; dysfonction et addiction psychosexuelles ; tolérance aux narcotiques ou sevrage de narcotiques ; augmentation du risque anesthésique, faculté de réponse aux anesthésiques ; dysfonctionnements hypothalamo-surrénaliens ; perturbations des rythmes biologiques et circadiens ; troubles du sommeil associés à des maladies telles que des affections neurologiques, y compris la douleur neurogène et le syndrome des jambes sans repos ; apnées du sommeil ; narcolepsie ; syndrome de fatigue chronique ; insomnies liées à des troubles psychiatriques ; tous les types d'insomnie idiopathique et de parasomnie ; perturbations du cycle sommeil-éveil, y compris syndrome du décalage horaire ; toutes les formes de démence et de dysfonction cognitive dans la population saine et chez des patients atteints de troubles psychiatriques et neurologiques ; dysfonctionnement mental lié au vieillissement ; tous les types d'amnésie ; arriération mentale profonde ; dyskinésies et affections musculaires ; spasticité musculaire, tremblements, troubles du mouvement ; dyskinésies spontanées ou d'origine médicamenteuse ; affections neurodégénératives, y compris chorée de Huntington, syndrome de Creutzfeld-Jacob, maladie d'Alzheimer et maladie de Gilles de La Tourette ; sclérose latérale amyotrophique ; maladie de Parkinson ; syndrome de Cushing ; lésions traumatiques ; traumatisme rachidien ; traumatisme crânien ; hypoxie périnatale ; baisse de l'acuité auditive ; tinnitus ; maladies démyélisantes ; affections des nerfs rachidiens et crâniens ; lésion oculaire ; rétinopathie ; épilepsie ; troubles convulsifs ; crises d'absence, convulsions complexes partielles et généralisées ; syndrome de Lennox-Gastaut ; migraine et céphalées ; affections douloureuses ; anesthésie et analgésie ; sensibilité accrue ou exagérée à la douleur, par ex. hyperalgésie, causalgie et allodynie ; douleur aiguë ; douleur des brûlés ; névralgie faciale atypique ; douleur neurogène ; dorsalgie ; syndrome douloureux régional complexe de type I et II ; douleur arthritique ; douleur due à une blessure sportive ; douleur dentaire ; douleur liée à une infection, par ex. au VIH ; douleur post-chimiothérapeutique ; douleur post-accident vasculaire cérébral ; douleur postopératoire ; névralgie ; arthrose ; pathologies associées à une douleur viscérale, par ex. syndrome du côlon irritable ; troubles du comportement alimentaire ; diabète ; syndromes toxiques et dysmétaboliques, y compris anorexie cérébrale , neuropathies diabétiques et alcoolisme ; troubles de l'appétit, du goût, du comportement alimentaire ou des apports de liquides ; troubles somatoformes, y compris hypochondrie ; vomissements/nausées ; épisodes émétiques ; dyskinésie gastrique ; ulcères gastriques ; syndrome de De Morsier-Kallman (anosmie); tolérance au glucose défectueuse ; dyskinésie intestinale avec anomalies de la motilité ; maladies hypothalamiques ; maladies hypophysaires ; syndromes d'hyperthermie, pyrexie, convulsions fébriles ; retard de croissance idiopathique ; nanisme ; gigantisme ; acromégalie ; adénome basophile ; prolactinome ; hyperprolactinémie ; tumeurs cérébrales, adénomes ; hypertrophie bénigne de la prostate, cancer de la prostate ; cancer de l'endomètre, du sein, du côlon ; tous les types de dysfonctionnement testiculaire, contrôle de la fertilité ; anomalies des hormones de la reproduction ; bouffées vasomotrices ; hypogonadisme hypothalamique, aménorrhée fonctionnelle ou psychogène ; incontinence urinaire d'origine vésicale ; asthme ; allergies ; tous les types de dermatite, d'acné et de maladies kystiques, dysfonctionnements des glandes sébacées ; maladies cardio-vasculaires; affections cardiaques et pulmonaires, insuffisance cardiaque aiguë et congestive ; hypotension ; hypertension ; dyslipidémies, hyperlipidémies, insulinorésistance ; rétention d'urine ; ostéoporose ; angor ; infarctus du myocarde ; arythmies, maladies coronariennes, hypertrophie ventriculaire gauche ; accident vasculaire cérébral ischémique ou hémorragique ; tous les types de maladies vasculaires cérébrales, y compris hémorragie sous-arachnoïdienne, accident vasculaire cérébral ischémique ou hémorragique et démence vasculaire ; insuffisance rénale chronique et autres maladies rénales ; goutte ; cancer du rein ; incontinence urinaire ; et autres pathologies liées à des dysfonctionnements généraux du système oréxine.

7. Utilisation d'un composé selon l'une quelconque des revendications 1 à 4 dans la préparation d'un médicament indiqué dans la prévention ou le traitement d'une affection sélectionnée dans le groupe consistant en les suivantes : tous les types de troubles du sommeil, de syndromes liés à un stress, d'usage ou d'abus de substances psychoactives, de dysfonctionnements cognitifs dans la population saine et chez des patients atteints de troubles psychiatriques et neurologiques, et de troubles du comportement alimentaire ou des apports de liquides.
